# EUROPEAN PATENT SPECIFICATION

(11) **EP 1 460 983 B1**
(45) Date of publication and mention of the grant of the patent: **02.10.2013**
(21) Application number: 02789097.9
(22) Date of filing: 25.11.2002
(51) Int. Cl.: A61F 13/472, A61F 13/47

(54) **ABSORBENT ARTICLE WITH IMPROVED FIT**
SAUGFÄHIGER ARTIKEL MIT VERBESSERTEM SITZ
ARTICLE ABSORBANT AVEC AJUSTEMENT AMELIORE

(30) Priority: 06.12.2001 SE 0104103
(43) Date of publication of application: 29.09.2004
(73) Proprietor: SCA Hygiene Products AB, 405 03 Göteborg (SE)
(72) Inventor: WIDLUND, Urban, S-435 43 PIXBO (SE)
(74) Representative: Olsson, Stefan
(86) International application number: PCT/SE2002/002151
(87) International publication number: WO 2003/047483

(56) References cited:
- EP-A1- 0 298 348
- EP-A2- 0 852 938
- EP-A2- 0 956 844
- EP-A2- 0 965 318
- WO-A1-99/25282
- US-A- 4 886 513
- US-B1- 6 210 385

## Description

### TECHNICAL FIELD

The present invention relates to an absorbent article, such as a sanitary towel, a panty liner, an incontinence pad, a nappy or the like, which article has a longitudinal direction and a transverse direction, a front portion, a rear portion, a crotch portion located between the rear portion and the front portion, an absorbent element and a liquidtight layer, and also a stiffening element which is intended to contribute to the three-dimensional shape of the article during its use.

### BACKGROUND ART

A great many different demands are made of absorbent articles, such as a sanitary towel, an incontinence pad, a nappy or the like, which are not easy to satisfy simultaneously. A fundamental requirement is that the article, for example a sanitary towel, should be capable of catching and absorbing bodily fluid discharged from the wearer. Conventional sanitary towels in sizes intended for heavy flows of menstrual fluid have been of thick and relatively wide design. Sanitary towels of this type are described in, for example, US 3 294 091. Thick and relatively wide sanitary towels of this type theoretically have great absorption capacity but in practice, when the sanitary towel is subjected to compression forces when squeezed together between the thighs of the wearer, much of the take-up capacity and absorption capacity is lost. The sanitary towel is squeezed together into an arbitrary rope-like shape which frequently does not offer a sufficiently large receiving surface for the menstrual fluid discharged, and leakage occurs in the case of heavy flows of menstrual fluid. The sanitary towel can also be pressed together between the thighs of the wearer in such a manner that the side edges of the sanitary towel and the liquidtight layer are folded in over the liquid-permeable surface and in this way reduce the size of the liquid-receiving surface available.

Sanitary towels are intended to be positioned inside a pair of briefs, the design of which may vary. In this connection, sanitary towels can be positioned incorrectly inside the briefs. There is therefore a risk of the sanitary towel being, by mistake, positioned too far forward or too far back or displaced slightly in the lateral direction and therefore of the absorption capacity and receiving surface of the entire sanitary towel not being optimally utilized.

Conventional sanitary towels are generally retained in the briefs of the wearer by means of pressure-sensitive adhesive or friction coatings. The sanitary towel is fitted by the towel being put in place in the briefs, after which the latter are pulled up into position. When fitting the article inside the briefs, however, it is difficult to achieve a positioning which is optimum in relation to the body of the wearer. Use is usually made of the crotch portion of the briefs in order to determine where the sanitary towel should be positioned. As sanitary towels are manufactured in a great many sizes and models, the position and design of the crotch portion provide a particularly uncertain indication of where in the briefs a sanitary towel is to be positioned, and the functioning of the sanitary towel during use is consequently not always as desired.

Another cause of leakage occurring past sanitary towels attached inside the briefs of the wearer is that the sanitary towel moves together with the briefs instead of following the body movements of the wearer. This means that even a sanitary towel which was from the outset positioned correctly in the briefs in relation to the body can be pulled out of this position by the briefs.

In order to attempt to reduce leakage arising as a result of the sanitary towel being pressed together between the legs of the wearer, it has become usual to provide the sanitary towels with special attachment flaps. It is known from, for example, SE 455 668, US 4 285 343, EP 0 130 848, EP 0 134 086 and US 4 608 047 to provide sanitary towels with flexible side flaps or wings projecting from the longitudinal side edges. These are intended to be folded around the edge portions of the briefs of the wearer when the sanitary towel is put on, and to be attached to the outside of the briefs. The side flaps themselves constitute protection against side edge leakage and soiling of the briefs. Moreover, deformation of the absorption body of the sanitary towel is counteracted by virtue of the fact that the sanitary towel is anchored at the leg edges of the briefs and is held extended between these during use.

However, a considerable disadvantage of providing absorbent articles with such attachment flaps is that many wearers find it embarrassing that the attachment flaps are visible on the outside of the briefs. This also means that absorbent articles with such attachment flaps cannot be used when, for example, the wearer is wearing a swimsuit.

Another disadvantage of the attachment flaps is that they are relatively difficult to handle and require many manual operations in order to be fitted correctly around the leg edges of the briefs. Furthermore, especially in the case of attachment flaps which extend quite a long way along the side edges of a sanitary towel, it can be virtually impossible to fold the attachment flaps around the curved leg edges of the briefs without chafing and unattractive creases in the attachment flaps occurring.

A further problem of sanitary towels with attachment flaps is that the functioning of the attachment flaps or wings depends on the design of the briefs. It goes without saying that a sanitary towel with attachment flaps interacts differently with briefs with a wide crotch compared with briefs with a very narrow crotch.

Attachment flaps or wings on sanitary towels protect the leg edges of the briefs from soiling but, as emerged above, are far from being an entirely satisfactory solution.

In order to improve leakproofness, EP 0 067 465 has proposed manufacturing a two-part sanitary towel in which the two parts are interconnected only at their end portions. The lower part is fastened in the briefs of the wearer, and the upper part makes contact with the body of the wearer. The idea is that the parts will be able to move slightly in relation to one another during use. The mobility between the parts is, however, very limited, and the known sanitary towel is still dependent on the movements of the briefs. Furthermore, there is no guarantee that the upper part will be held in contact with the body of the wearer during use.

PCT/SE96/01061 describes another two-part absorbent article in which the two parts are movable in relation to one another. This known article also has limited mobility between the parts and is to a certain extent dependent on the movements of the briefs.

One way of attempting to reduce the risk of edge leakage caused by deformation of the sanitary towel during use is to provide the sanitary towel with a preshaped raised portion, what is known as a hump, which is intended to make contact with the genitals of the wearer during use of the sanitary towel. Discharged bodily fluid can in this way be caught as soon as it leaves the body of the wearer and be absorbed immediately into the article instead of running out over the surface of the latter. A raised portion also makes it easier for the wearer to position the article correctly in relation to the body. French patent publication FR-A-2 653 328 describes a sanitary towel with a hump in the form of a central, longitudinal, cylindrical raised portion.

A common way of creating a raised portion has been quite simply to build it up by arranging a greater quantity of absorption material within the area of the raised portion. As the absorption material used is in most cases what is known as cellulose fluff pulp, however, such a raised portion collapses and loses its shape when it is wetted. In order to produce a raised portion which is sufficiently large in the wet state as well, a raised portion consisting of cellulose fluff pulp must comprise so much absorption material that it is altogether too high, hard and uncomfortable to wear in the dry state.

It is also known to produce an article with a raised portion facing the wearer by positioning a shaping element on top of the absorbent core. The disadvantage is that this interferes with the liquid transport down to the absorbent, liquid-retaining absorption core and that leakage can occur because the shaping element does not have sufficient admission capacity or temporary retention capacity. The use of, for example, a foamed material in the raised portion has been proposed. However, it has proved difficult to produce a foamed structure with sufficiently open pores for good liquid admission into the latter at the same time as the material is to have such great retention capacity that liquid is not pressed out in the event of loading originating from the wearer, for example when the latter sits down.

Another example of a raised portion is described in Swedish patent 507 798. Such a raised portion has a predictable shape, both before and during use, and also keeps its shape irrespective of the movements of the wearer and of the wetting to which it is subjected. The raised portion is anatomically designed, which means that it is relatively narrow in order to project in slightly between the labia of the wearer during use without causing discomfort for the wearer.

Although such a raised portion functions well for its purpose, it has been found that when the raised portion is exposed to large quantities of bodily fluid over a relatively short period of time, there is a risk that some of the liquid will run on the outside of the raised portion and flow out past the side edges of the absorbent article. Such leakage can occur, for example, when the wearer of a sanitary towel has been sitting or lying down for a relatively long period of time and then suddenly rises. This is because, when the wearer is sitting or lying down, a relatively large quantity of menstrual fluid accumulates in the vagina of the wearer. In the event of a sudden change in body position, the entire quantity of accumulated liquid may be discharged at once. A narrow raised portion of the type described in SE 507 798 does not then have a sufficiently large surface to be capable of receiving and absorbing the entire quantity of liquid in one go, for which reason such sudden liquid flows often result in leakage.

EP 0 335 252 and EP 0 335 253 have proposed providing an absorbent article with a deformation element. The deformation element is acted on by the transverse compressive forces between the thighs of a wearer. The purpose of the deformation element is to cause a portion of the article to bulge in the direction of the body of the wearer during use. It is impossible, however, to control or predict entirely the shape the article will adopt for each individual wearer. Moreover, it is not possible to ensure contact between the body of the wearer and the surface of the article, because the degree of bulging is determined entirely by how much the article is compressed in the transverse direction.

US 4 804 380 describes an absorbent article which has a permanent three-dimensional shape. The article has one end portion of flat or concave shape and one end portion provided with a raised portion. The flat or concave end portion is intended to be positioned in front of the mons Veneris of the wearer, and the end portion comprising the raised portion is intended to fit in against the buttocks of the wearer. The three-dimensional design of the article is brought about by folding a fairly stiff absorption body. In order to make the raised portion permanent, the rear side of the article is provided with a glued surface in the end portion which is to have the raised portion. When the raised portion has been formed, it is maintained by means of the glue.

There are absorbent articles on the market which have a permanent, three-dimensional, boat-like shape and in which the outer shell consists of a moulded polymer foam.

A considerable disadvantage of permanent three-dimensional products is that it is difficult to pack a stiff three-dimensional product. Such products require a great deal of space for transport and sale, and it can be embarrassing for a wearer to carry around a sanitary towel or an incontinence pad which it is impossible to fold and therefore cannot be concealed in the hand or in the worst case will not even fit in a handbag.

EP 155 515 describes how an absorbent article, such as a sanitary towel, is provided with a bowl-shaped appearance by virtue of elastic being applied in a pretensioned state at the longitudinal side edges of the article. The use of elastic complicates manufacture, and there is a risk of the intended elastic effect being lost in connection with packing of the article or when the latter is stored in a folded packing state.

It is previously known to design plane absorbent articles which adopt a three-dimensional, essentially bowl-like shape when applied. An example of this is described in US 4 655 759. This discloses an elongate sanitary towel which consists of a layer of absorbent material, a flexible liquidtight outer layer and a liquid-permeable inner layer. The sanitary towel is provided with a pair of channels formed by stamping, the channels being located on both sides of a longitudinal centre axis and extending along a curved path over the absorption material layer. The two paths together form an hourglass-like shape positioned centrally over the towel. Before use, the sanitary towels are essentially plane but, when they are applied to the wearer, they are folded into a bowl-like shape, that is to say with liquid-stopping upright borders outside the channels. One disadvantage of this bowl-like construction is that the borders hold the central portion of the sanitary towel at a distance from the genitals of the wearer, and liquid discharged from the wearer does not flow directly into the absorbent article but can run on the surface, the risk then being obvious that liquid may find an undesirable transport path in the form of a small crease or the like and run straight out of the product in the lateral or longitudinal direction. Stamped channels in an absorption body also have the disadvantage that the liquid spread in the absorption layer is disrupted and that absorption material outside the channels is not utilized, which increases the risk of local oversaturation and attendant leakage from those parts of the absorption layer which are used.

Previously known sanitary towels and the various problems associated with them have in the main been discussed above. However, what has been said above also applies to incontinence pads. Nappies for children and adults also belong to the same problem area as far as fit in the crotch and take-up of liquid in an absorption body are concerned.

As emerged above, great efforts have been made over many years in order to attempt to solve all the problems associated with absorbent articles, such as sanitary towels. Although great improvements have been made, all the previously known solutions are associated with some disadvantages.

### DISCLOSURE OF INVENTION

By means of the present invention, an improved absorbent article of the type mentioned in the introduction has been produced. The article according to the invention is characterized mainly in that the stiffening element is in a plane state before use of the article, in that the stiffening element is provided with at least one first cutout which is arranged so as to make possible when the article is in use pressing-together of the stiffening element in the transverse direction at a transition between the front portion and the crotch portion, in that the shape of the stiffening element and the shape of the first cutout are adapted so that the stiffening element, after pressing-together and positioning on the wearer, has a width at said transition which is adapted to the distance between the muscle tendons of the wearer on both sides of the crotch of the wearer in the groin of the latter and which width is of the order of 15-35 mm, in that the stiffening element is arranged so that its side edges in the front portion of the article, in said pressed-together utilization state, can diverge in the direction from the crotch portion at least part of the way in over the front portion, and can form an acute angle with a line in the longitudinal direction of the article, in that, in the plane state before use the stiffening element has a width in the transition between the front portion and the crotch portion which exceeds 45 mm, and in that the stiffening element is absorbent.

An absorbent article according to the invention has a number of advantages. It is plane before use, and there are therefore no problems associated with packing, storing and transporting said article. An absorbent article according to the invention automatically adopts a three-dimensional bowl-like shape in an area in the front portion next to the crotch portion when the article is, at its transition between the front portion and the crotch portion, pressed together in the lateral direction at the weakening created by the cutout and fixed in between said muscle tendons. It is known that the distance between said muscle tendons is very similar for all people. Fatness of course has an effect on the width between the thighs, but the width between the muscle groups is the same, and it is these which may cause an article to feel as if it chafes. The fat tissue lies on the outside of the muscles but does not contribute to any sensation of discomfort. The distance between said muscle tendons is the same irrespective of whether the wearer is slim, of normal weight or overweight. It has been found that what determines whether a wearer experiences discomfort in the form of pressure or chafing against the insides of the thighs is whether the absorbent article has a width during use which in the critical area considerably exceeds the distance between the muscle tendons in the groin portion. This distance has been found to be roughly 25-45 mm. It has been found that an article with a width which exceeds 40 mm in the critical area during use feels uncomfortable to wear to the majority of wearers. On the other hand, it is rarely experienced as being unpleasant if an absorbent article pushes down or aside fat tissue which may be present in the crotch area of the wearer.

Surprisingly, it has been found that this distance between said muscle tendons does not change throughout the lifetime of a person. Small infants therefore have a corresponding critical distance, which, according to the present invention, can be utilized for producing nappies for infants with an improved fit. The same of course applies for nappies for adults. It should be pointed out that said critical distance between the muscle tendons applies for , men also, who have the same distance between said muscle tendons.

An article designed according to the invention is adapted to the anatomy of the wearer. The special geometry around the transition between the crotch portion and the front portion results in an article being pressed together at said transition during use and anchored firmly in the groins of the wearer, and in this way the article is prevented from moving backwards between the legs of the wearer: This is otherwise a common problem in conventional articles because the leg movements of the wearer often shift the article backwards.

According to one embodiment, the invention is characterized in that the stiffening element extends in the longitudinal direction of the article over at least part of the front portion, and in that the first cutout is symmetrical in relation to a centre line along the longitudinal centre line of the article and extends over at least a portion of the front portion.

According to one embodiment, the invention is characterized in that the stiffening element (6) at the same time constitutes the absorbent element, and in that, during absorption in the pressed-together utilization state, it is adapted so as to swell while on the whole retaining its geometry in the transverse direction and longitudinal direction of the article.

According to one embodiment, the invention is characterized in that, in the pressed-together utilization state, said width of the stiffening element at the transition between the crotch portion and the front portion is of the order of 20-35 mm.

According to another embodiment, the invention is characterized in that, in the pressed-together utilization state, said width of the stiffening element at the transition between the crotch portion and the front portion is of the order of 25-30 mm.

It has been found that a width of 30-32 mm at said transition fits well for of the order of 80% of all wearers and that said width of the stiffening means should therefore fit virtually all wearers without risk of chafing.

The stiffening element suitably has a stiffness of in the order of 1-15 N measured according to ASTM D 4032-82. This "Circular Bend Procedure" is described in detail in EP 336 578.

According to one embodiment, the invention is characterized in that the stiffening element consists of a dry-formed fibre mat with a density between 0.15 and 0.75 g/cm³ and a weight per unit area of in the order of 100-400 g/m².

A dry-formed fibre mat of this kind is described in US 5 730 737. The fibre mat produced is very stiff after forming and compression. The fibre mat can be used as it is or be mechanically softened to the desired stiffness.

A way of very accurately forming fibrous webs for use as absorption elements in absorbent articles is described in Swedish patent application 0101393-7. The fibrous webs are formed by air-laying fibres, separate air flows containing fibres being fed to a number n of different mat-forming wheels, where n is a whole number which is at least 2. Separate web layers are formed on the individual web-forming wheels. The fibrous web is formed by said web layers being combined to form a common fibrous web downstream of the mat-forming wheels, which web has very great manufacturing accuracy by virtue of the manufacturing method. The manufacturing speed and thus the web speed can be very high, and the desired manufacturing accuracy at the web speed concerned is achieved by selecting a sufficiently high number n of mat-forming wheels. By virtue of this manufacturing method, very thin fibrous webs can be manufactured with very great accuracy.

According to one embodiment, the invention is characterized in that the dry-formed fibre mat is provided with the desired stiffness and the desired extensibility by virtue of the degree of compression selected and the compression pattern selected.

According to one embodiment, the article according to the invention is characterized in that the side edges of the stiffening element, which, in the pressed-together utilization state of the article, diverge at least part of the way from the crotch portion in over the front portion of the article, are arranged so as in this state to form an angle between a line in the longitudinal direction of the article and each of said side edges of in the order of 35-55°, preferably in the order of 45°. With this geometry in and around the transition between the crotch portion and the front portion, effective anchoring is obtained without the wearer experiencing any discomfort in the form of chafing or the like.

According to one embodiment, the article according to the invention is characterized in that the stiffening element also extends part of the way in over the rear portion of the article, and in that the stiffening element is arranged so that its side edges, in the pressed-together utilization state of the article, diverge in the direction from the crotch portion at least part of the way from the crotch portion in over the rear portion of the article. The crotch portion suitably has a length of between 70 and 120 mm. This length corresponds to the length of a plane portion in the crotch portion of a woman. The stiffening element according to the last embodiment is therefore anchored both at the front and at the rear at the transition between the crotch portion and the front portion and, respectively, at the transition between the crotch portion and the rear portion, as a result of which an article which is very stable, well fixed and at the same time comfortable during use is obtained.

According to one embodiment, the article according to the invention is characterized in that the stiffening element, seen in the longitudinal direction of the article, comprises a first, front stiffening part element which, in the pressed-together utilization state, has said width at the transition between the front portion and the crotch portion, and at least one further, second stiffening part element which is separate from the first part element at said transition, as a result of which the first and the second part element are movable in relation to one another.

According to one embodiment, the invention is characterized in that the second stiffening part element also extends part of the way in over the rear portion of the article, and in that, in the pressed-together utilization state of the article, the side edges of the second stiffening element are arranged so as, in the direction from the crotch portion, to diverge at least part of the way from the crotch portion in over the rear portion of the article.

According to another embodiment, the invention is characterized in that the stiffening element extends over said transition, and at least part of the way over the front portion and also at least part of the way over the crotch portion, and in that the first cutout consists of at least one hole which is widest at least directly in front of the transition and is circular or oblong.

According to one embodiment, the stiffening element 6 has, in the plane state before use, the same width along its length with the exception of the end portions which suitably are rounded.

According to one embodiment of the invention, the first cutout consists of a cutout open towards the transition.

According to another embodiment, the first cutout is in its entirety located in the front portion and is essentially diamond-shaped.

According to another embodiment, the invention is characterized in that a hump-forming element made of a resilient material is arranged under the stiffening element over at least part of the crotch portion, which hump-forming element is arranged so as to bring about a raised portion on the side which is intended to be fitted against the wearer, the raised portion being arranged so as to come to lie directly in front of the genitals of the wearer after fitting of the article on the wearer.

According to one embodiment, the article according to the invention is characterized in that the stiffening element serves as an absorption means and has very great liquid-spreading capacity for spreading bodily fluid received in the relatively narrow crotch area bounded by the anatomy of the wearer directly in front of the genitals of the wearer over the absorbent portions of the whole article, and in that the stiffening element is designed with great swelling capacity in the depth direction and attendant great absorption capacity.

Further embodiments of the article according to the invention emerge from the subsequent patent claims.

### DESCRIPTION OF FIGURES

The invention will be described in greater detail below with reference to illustrative embodiments shown in the accompanying drawings, in which:
- Figure 1: shows a plan view of an absorbent article according to a first embodiment;
- Figure 2: shows the article according to Figure 1 in a pressed-together utilization state;
- Figure 3: shows a section along the line III-III in Figure 2 in a curved utilization state;
- Figure 4: shows a plan view of an article according to a second embodiment;
- Figure 5: shows the article according to Figure 4 in a pressed-together utilization state;
- Figure 6: shows a section along the line VI-VI in Figure 5;
- Figure 7: shows a plan view of a third embodiment of the article according to the invention;
- Figure 8: shows a plan view of a fourth embodiment of the article according to the invention seen towards that surface of the article which receives bodily fluids;
- Figure 9: shows a plan view of a fifth embodiment of the article according to the invention seen towards that surface of the article which receives bodily fluids;
- Figure 10: shows a plan view of the article according to Figure 9 from the opposite side;
- Figure 11: shows a section along the line XI-XI in Figure 9 but in a curved utilization state;
- Figure 12: shows, in perspective and in a utilization state, the article according to the fifth embodiment and also the embodiment shown in Figures 9-11;
- Figures 13-16: show diagrammatically plan views of four further embodiments of the article according to the invention, and
- Figure 17: shows a plan view of an absorbent article according to the invention and in the form of a nappy.

### MODE(S) FOR CARRYING OUT THE INVENTION

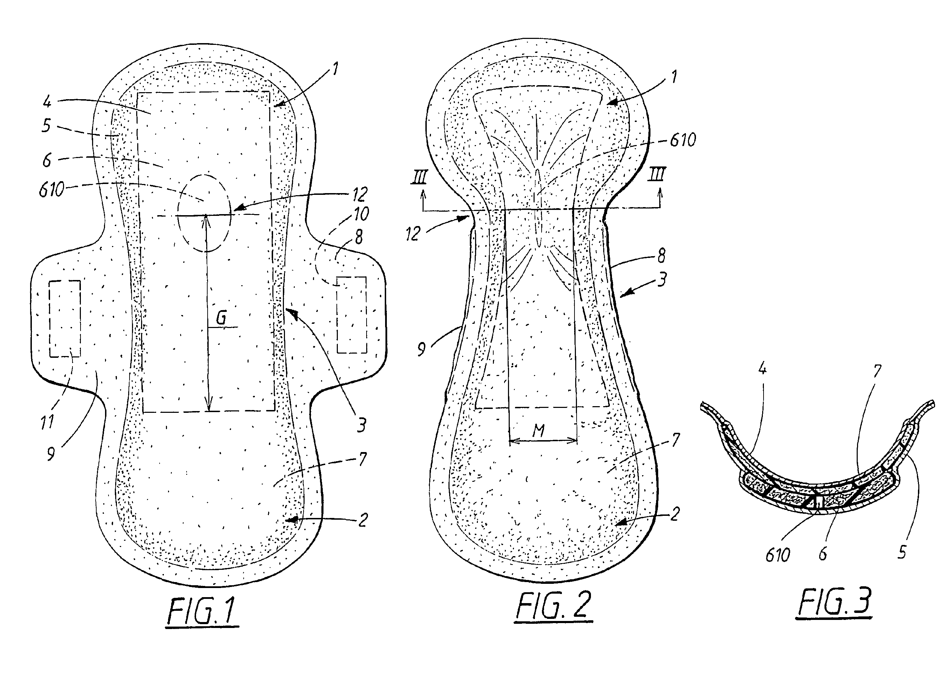

Figures 1-3 show an article according to the invention in the form of a sanitary towel or incontinence pad. The article is elongate with a longitudinal direction and a transverse direction. The article has a front portion 1, a rear portion 2 and a crotch portion 3 located between said portions. The article shown in Figures 1 to 3 comprises a liquid-permeable inner layer 4 which is intended to face the wearer during use of the article. The inner layer, which makes contact directly with the skin of the wearer, is suitably made from a soft, textile-like material. Examples of suitable liquid-permeable materials are various types of what are known as non-woven fabrics. Other examples of suitable materials are perforated plastic films. Net and knitted or woven textiles as well as combinations and laminates of said materials can also be used as the inner layer. Examples of inner layers for sanitary towels are laminates of various non-wovens and laminates of non-wovens and perforated plastic films. The liquid-permeable layer can also be integrated with underlying drainage or absorption layers; for example a foam plastic with open pores and with a density gradient in the depth direction can serve as a surface layer and as a drainage layer and/or absorption layer.

The absorbent article also has a liquidtight outer layer 5. This usually consists of a thin plastic layer, made of polyethylene for example. It is also possible to use a liquid-permeable material which has been treated with hydrophobing agent in order to make it liquidtight. In particular if the absorbent article is relatively large, it may be suitable for the outer layer to be vapour-permeable in addition to being liquidtight. Such layers can consist of hydrophobed non-woven fabric or of porous plastic films.

The absorbent article includes an absorbent element 6 of rectangular shape, and a liquid-permeable insulating layer 7 which has a keyhole-like shape with a greater extent in both the longitudinal direction and the transverse direction than the absorbent element 6. The outer layer 5 and the inner layer 4 extend with edge portions outside the insulating layer around the latter and are interconnected along these edge portions to form a cover around the absorbent element 6 and the insulating layer 7. In the region of the crotch portion 3, the cover formed by the inner and outer layers extends outwards in the lateral direction to form flexible side flaps 8, 9, what are known as wings, which are intended to be arranged around the crotch portion on the briefs of the wearer in order to protect the edge portions of the briefs from soiling. The wings 8, 9 are suitably provided with adhesive coating, which has been indicated in Figure 1 by reference numbers 10, 11, on the outer layer 5, by means of which the wings can be attached around the legs of the briefs. As can be seen from Figure 2, the insulating layer 7 is located directly inside the inner layer 4 and is principally intended for rapidly admitting discharged bodily fluid into the underlying absorbent element 6 and forming a liquid-insulating layer so as to reduce what is known as back-wetting from the absorbent element 6 to the inner layer 4 making contact directly with the wearer.

The insulating layer can consist of, for example, an airlaid fibrous material of low density bonded together with bonding agent or thermofibre, which is marketed under the designation LDA (low density airlaid). The absorbent element 6 is, seen from the liquid-permeable inner layer 4, arranged under the insulating layer 7. In the illustrative embodiment shown here of the article according to the invention, this element is designed to take up and retain essentially all the bodily fluid discharged. The absorbent element 6 has smaller capillaries than the insulating layer 7 located above and therefore draws liquid from the insulating layer and prevents back-wetting by liquid from the absorbent element to the insulating element and to the inner layer 4 which remains essentially dry during use of the article. Only when the absorbent element is saturated with liquid can transport take place from the absorbent element to the insulating layer.

The liquid-insulating layer 7 and the absorbent element 6 can of course be made from materials other than those indicated above. The important aspect is that the absorbent element 6 has greater liquid-affinity than the liquid-insulating layer 7 so that liquid is transported from the insulating layer to the absorbent element but not vice versa.

The liquid-insulating layer can consist of, for example, what is known as a multibond non-woven, that is to say a non-woven fabric in which fibres are bonded by both bonding agent and melt bonds. This can also contain fibres or particles made of a slow-acting superabsorbent material and/or an odour-inhibiting superabsorbent material.

In the illustrative embodiment shown, the absorbent element 6 is also intended to serve as a stiffening element and is to this end designed so as to be very stiff in order as far as possible to avoid the absorbent article being compressed in an uncontrolled manner when squeezing forces in the lateral direction occur, generated by the thighs of the wearer in the crotch area. The absorbent stiffening element has a size, shape and stiffness which result in the article, throughout its time of use, retaining a predetermined shape and moreover being retained in the intended position on the wearer.

In this context, the expression stiffening area means that an area has been reinforced in some way in order that this area is stiffer than the rest of the article. This reinforcement can consist of a separate reinforcing element which, as in the embodiment according to Figures 1 and 2, also serves as an absorbent element, or a completely separate stiffening element which has only a stiffening function and can consist of an element, made of paper or plastic for example, which is stiff in relation to the rest of the article and can be constructed from one or more material layers made of the same material or different materials. Alternatively, the stiffening area can be brought about by virtue of the article having been stiffened in this area by extra bonding agent between individual material plies. Alternatively, the article can consist of material which is permanently compressible at least in the area which is to be stiffened, suitable compression taking place during manufacture of the article to bring about the desired stiffness in the area concerned. The latter illustrative embodiment is described in greater detail below.

In the description below, the expressions stiffening area and stiffening element will be used interchangeably, the most suitable expression being selected in order to clarify what is meant at the point concerned in the text.

As can be seen from Figure 1, the absorbent stiffening element 6 extends over the front portion 1 and the crotch portion 3.

The stiffening element 6 is rectangular and, at the transition 12 between the crotch portion 3 and the front portion 1, has an elongate through-hole 610. This hole is intended to make it possible for the stiffening element 6 to be pressed together in the lateral direction, which pressing-together is at a maximum where the hole is widest when pressing-together takes place. During pressing-together, curvature of the article also takes place, as can be seen most clearly from Figure 3. After pressing-together, the stiffening element 6 has a width M during use which is adapted to the distance between two particular muscle tendons on both sides of the crotch of the wearer directly in front of the groins. These muscle tendons form part of the muscle group which originates on the inside of the pelvic diaphragm and has its attachment along the thigh. This muscle group consists of the adductor brevis, adductor longus, gracilis and adductor magnus muscles. As mentioned above, it is known that this distance between said muscle tendons is very similar for all people. This dimension is of the order of 25-45 mm. Research has shown that 80% of all women have a dimension of roughly 30-32 mm between said muscle tendons. When said width M essentially corresponds to the distance between said muscle tendons on the wearer, the article will be anchored firmly during use with the transition portion between the muscle tendons and be retained in this position.

As can be seen from the above, it is the through-hole 610 which makes it possible to press the article together at the transition 12. By virtue of the selection of the stiffness of the stiffening element, the shape and size of the hole and also the material width of the stiffening element in the area directly in front of the hole, the desired resilience can be obtained when the article is pressed together. The result of this is that an article of a certain size can fit wearers with a slightly different width between said muscle tendons, that is to say the hole 610 can be pressed together completely or partly during use of the article.

After pressing-together of the article, the two side edges of the front portion diverge in the forward direction on the article from said transition area 12. In this way, the article is prevented from moving backwards between the legs of the wearer. This is otherwise a common problem in conventional sanitary towels because the leg movements of the wearer often shift the sanitary towel backwards.

In Figure 2, an angle between a line in the longitudinal direction of the article and each of said side edges has been designated by α. In the case of a large angle α, for example close to 90°, the edges of the front portion may chafe against the groins and legs of the wearer and in this way cause discomfort for the wearer. The smaller the angle α after pressing-together of the article directly in front of the hole 610 during use, the greater the risk that the article will slide backwards in between the legs of the wearer. In the case of an angle of less than 30°, this risk is unacceptably high. An angle of 35-45° provides the best balance between secure positioning and comfort. An angle of roughly 45° has been found to be especially favourable.

An absorbent article, such as a sanitary towel, according to the invention is designed with a crotch length adapted to the anatomy of the wearer. In a sanitary towel according to the invention, use has been made of the fact that the great majority of women have a crotch length of in the order of 80-100 mm. The stiffening element 6 has therefore been designed with a corresponding crotch length G of in the order of 70-120 mm, that is to say the distance from the transition area 12 to the start of the rear portion. In the embodiment according to Figures 1-3, the transition area 12 is defined as an area around the centre of the hole 610.

Along the crotch, where the body shape of the wearer is essentially plane, the sanitary towel according to the invention is designed so as in the dry state to be relatively stiff in the lateral direction, that is to say it is sufficiently stiff not to be deformed in an uncontrolled manner in the lateral direction and form creases. As the stiffening element 6 in the embodiment described here also constitutes the major part of the absorption capacity of the sanitary towel, it is essential for it to be possible to utilize available space between the legs of the wearer in the crotch. The width of the sanitary towel in the crotch area is, with regard to the stiffening element, limited at the front by said distance between said muscle tendons directly in front of the groins of the wearer. In the backward direction from said transition area to the end of the crotch portion, the width of the stiffening element 6 and thus the absorbent element can increase continuously to of the order of 1.5 times the width in the transition area 12 between the crotch portion and the front portion without any risk of the stiffening element chafing the wearer in the crotch. The size and shape obtained in the crotch area is determined by the width and length of the stiffening element and the shape and extent of the hole.

The abovementioned geometrical design of the area in and around the transition area 12, that is to say the size of the angle α and the width M, which is varied by means of the width of the stiffening element and the width of the hole together with the stiffness of the stiffening element as mentioned above, and also the selected crotch length G on the stiffening element for the article according to the invention, affords a very good anatomical adaptation of the stiffening element, which gives the article a good fit and stability in the fitted position on the wearer. This is of particularly great importance for the functioning of the article, not least because the wetting point can, on account of the body position of the genitals of the wearer in the longitudinal direction of the crotch area, vary by as much as of the order of 20 mm for different wearers. As the available space around the wetting point is very limited in width and length, optimum positioning and anchoring in this position of the stiffening absorbent element is necessary. This is achieved by means of said distances M and G and said angle α.

The anchoring effect is achieved at said muscle tendons even when, after the article has been pressed together during use, the width M on the article is slightly less than the distance between said muscle tendons directly in front of the groins. The two edge portions of the front portion diverge in the forward direction, and the article can slide backwards slightly until the edge portions are anchored firmly between said muscle tendons. The distance M on the article is suitably of the order of 15-35 mm and preferably 25-30 mm. The latter distance fits most wearers. If, after pressing-together, the distance exceeds roughly 35 mm, articles may therefore feel uncomfortable to some wearers. A distance in excess of 45 mm is unsuitable because such articles cause discomfort in the form of chafing for most wearers.

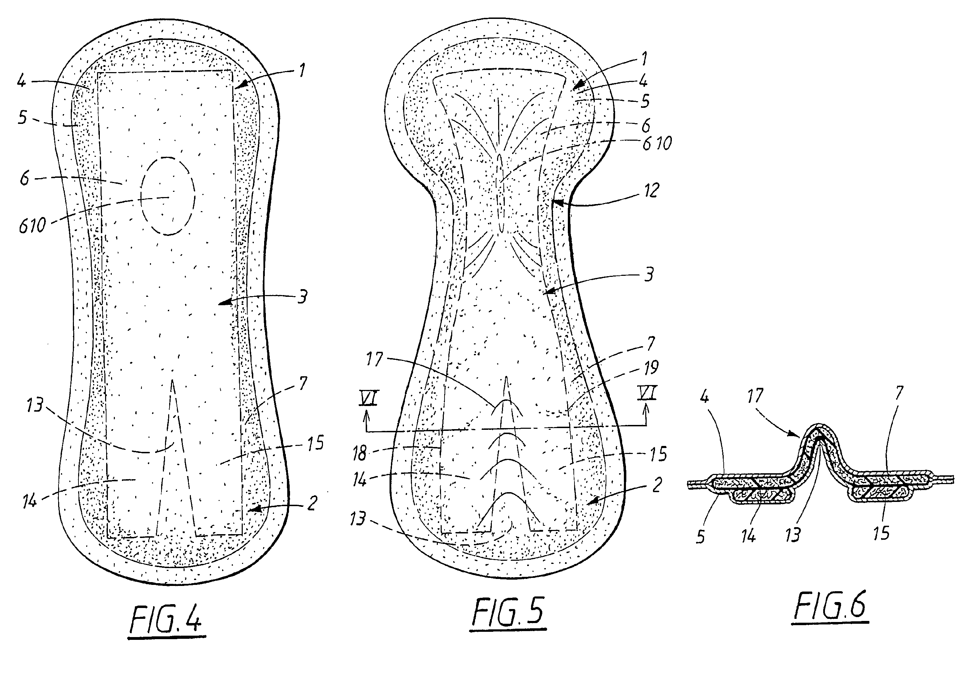

In the embodiment according to Figures 4-6, the stiffening element 6 also extends part of the way in over the rear portion 2 of the article. In the rear portion, the stiffening element has a cutout 13 extending from its end edge in the direction towards the crotch portion, as a result of which the article can fold along a longitudinal line L in the cutout and as a result of which the stiffening element forms legs 14 and 15 which are located on both sides of the cutout and are more flexible than the wider crotch portion. The legs 14 and 15 can be made vertically movable in relation to one another by virtue of the width selected for the cutout. This cutout 13 is very important for the adaptation and flexibility of the article in relation to the body. The fold formed in the cutout during use of the article can penetrate the cleft between the buttocks of the wearer and in this way provides very good protection against leakage via the cleft between the buttocks, which type of leakage usually occurs during the use of conventional products when the wearer is lying on her back. The cutout 13 also makes it possible for said legs 14, 15 of the stiffening element to be displaced vertically in relation to one another when various body movements take place, for example when the wearer is walking.

In the illustrative embodiment shown in Figures 4-6, the cutout 13 is wedge-shaped and located symmetrically in relation to the longitudinal symmetry line L of the article and also forms an angle β of in the order of 20°. This angle can vary within wide limits but of course depends on the design of the rear portion 2. When the rear portion is of considerably wider design, said angle β can vary between of the order of 10° and 120°, preferably between 15° and 40°.

In the illustrative embodiment shown, the stiffening element 6 also serves as the main absorption element of the article and has very great liquid-spreading capacity for rapid spreading of bodily fluid received from the wearer in the narrow crotch area directly in front of the genitals of the wearer over the absorbent portions of the whole article, that is to say over the entire stiffening and also liquid-absorbing element 6. This stiffening absorbent element is designed so as to swell in the depth direction during absorption and on the whole retain its geometry in the transverse direction of the article, which results in the stiffening element retaining its fit and secure positioning in relation to the body of the wearer throughout use of the article. The absorbent stiffening element 6 has great swelling capacity in the depth direction and attendant great absorption capacity.

According to a suitable embodiment, the stiffening absorbent element 6 consists of a dry-formed fibre mat with a density between 0.15 and 0.75 g/cm³ and a weight per unit area of in the order of 100-400 g/m². A dry-formed fibrous mass in the form of a fibre mat is described in US 5 730 737. The fibre mat produced is very stiff after forming and compression. The fibre mat can be used as it is or be mechanically softened to the desired stiffness.

A way of very accurately forming fibrous webs for use as absorption elements in absorbent articles is described in Swedish patent application 0101393-7. The fibrous webs are formed by air-laying fibres, separate air flows containing fibres being fed to a number n of different mat-forming wheels, where n is a whole number which is at least 2. Separate web layers are formed on the individual web-forming wheels. The fibrous web is formed by said web layers being combined to form a common fibrous web downstream of the mat-forming wheels, which web has very great manufacturing accuracy by virtue of the manufacturing method.

The manufacturing speed and thus the web speed can be very high, and the desired manufacturing accuracy at the web speed concerned is achieved by selecting a sufficiently high number n of mat-forming wheels. By virtue of this manufacturing method, very thin fibrous webs can be manufactured with very great accuracy.

The fibre mat for forming the stiffening absorbent element can consist of a mixture of cellulose fibres and viscose fibres, the presence of the latter giving the fibre mat a greater wet strength than a fibre mat made of only cellulose fibres. The fibre mat for forming the stiffening absorbent element can also contain synthetic melt fibres, by means of which the strength of the fibre mat can be increased by heat treatment to melt said synthetic melt fibres.

A further example of stiffening absorbent material is a laminate in the form of one or more plies of tissue and superabsorbent material (SAPs). The material or combination of different materials serving as an absorbent element and also, if appropriate, as a stiffening element can contain SAPs in the form of fibres, particles or foam.

The selection of compression pattern also makes it possible to vary the extensibility of the fibre mat. The dry-formed fibre mat can be provided with the desired reduced stiffness and the desired extensibility by virtue of the degree of compression selected and the compression pattern selected.

Furthermore, it is possible to pattern-compress only specific zones for the purpose of providing only these zones with an extensibility and stiffness which are different from the rest of the stiffening absorption element. In the same way, the stiffening absorption element can be compressed over its entire extent but with different patterns in different zones. By virtue of the presence of a stiffening absorption element which can in a simple manner, by virtue of the pattern compression selected, be provided with the desired stiffness and the desired extension in different zones, and in which the stiffness and extension properties can be selected essentially freely in these zones, the present invention has brought about a new and previously unknown way of controlling and guiding the shaping of an absorbent article intended for taking up bodily fluids.

As mentioned above, the stiffening absorbent element 6 has great swelling capacity in the depth direction, which, when a dry-formed fibre mat as above is used, has been achieved by great compression of the fibre mat in connection with its production. In the dry state, the fibre mat is hard-compressed and stiff, which affords the shaped and anatomically adapted absorption element very good stability in the fitted position on the wearer and very great spreading capacity, as a result of which the total absorption capacity of the absorption element can be optimally utilized and leakage caused by local oversaturation can to a great extent be eliminated. During absorption of liquid, the absorption body swells mainly in the depth direction but the absorption element does of course swell slightly in other directions as well. When the anatomically adapted stiffening absorption element swells, further improved anatomical adaptation is in fact achieved, which contributes to the stability and flexibility of the article in relation to the body shape of the wearer when the stiffness of the absorption element decreases during absorption and attendant swelling.

So as to function in the desired manner, the stiffening element has a stiffness in the dry state of in the order of 1-15 N measured according to ASTM D 4032-82. This "Circular Bend Procedure" is described in detail in EP 336 578.

The stiffening absorbent element can consist of a laminate of a number of non-woven fabric layers or tissue layers which are fixed to one another for increased stiffness and which have highly absorbent particles between individual plies. The individual plies can be fixed to one another by a bonding agent, such as adhesive or melt fibres. The highly absorbent particles can also contribute to bonding. The stiffness is controlled by virtue of the selection of the number of plies and the quantity of bonding agent included and the selection of highly absorbent material and how the adhesive capacity thereof is utilized.

A stiffening absorbent element of this type can also be provided with different stiffness and different extensibility in different zones of the extent of the element. These properties can in this case as well be controlled by means of compression patterns. This compression can be combined with the supply of heat, which supply can vary in different zones. Furthermore, bonding agent can be applied in different patterns to control the shaping of the stiffening absorption element during use. A varying supply of moisture in different areas in connection with compression is another parameter for controlling the shaping of the article during use.

Another example of the construction of a unit serving as both absorption element and stiffening element is a number of layers of LDA, that is to say layers of the same type as in the drainage and insulating layer 7. However, the bonding of the layers of LDA in the stiffening absorption element is much harder both within and between individual layers. This bonding is brought about by hard-compression of the LDA layers and suitably by using both melt fibres and latex, what is known as the multibond technique. In this design as well, stiffness and extensibility can be controlled by compression pattern selection and also by variation of the heat supply in different zones.

Further material examples are mixtures of LDA and HDA (high density airlaid) if appropriate in combination with other material layers, such as tissue.

Pattern compression can be used in all the material examples described above, and it is then possible to achieve, for example, hinge effects along compression lines or compression zones.

Pattern formation can take place in connection with compression of the stiffening absorption element. Alternatively, pattern compression can take place in a separate step after smooth compression. Use can be made of, for example, a web of material made in one of the ways described above and smooth-compressed as the starting material for the stiffening absorption element, which is pattern-compressed in the desired manner and depending on the type and size of article to be manufactured. After pattern-compression, individual products are cut out. Pattern-compression and cutting-out of separate stiffening absorption elements can take place in a single step in a combined cutting and pattern-compression unit.

As described above, the stiffening element can also constitute the main absorption element of the article. This is suitable from the point of view of production because there are fewer elements to handle than if, for example, the stiffening element and the absorption element constitute separate elements.

The invention also comprises designs in which the stiffening element is separate from the main absorption element of the article. The stiffening element can then be absorbent or non-absorbent. The main purpose in such a design is to constitute a stiffening shaping element.

In addition to the interpretation of the term stiffening element as constituting a completely separate element or constituting both the main absorption element and the stiffening element of the article, the term can also embrace the interpretation that all the material plies, bonding agents etc. included in the article in the area of the desired stiffening together form the desired stiffening element. In such a design, the expression stiffening area can also suitably be used instead of stiffening element. Absorption element and stiffening area can be made of one and the same material, for example a foamed material or a body constructed from fibres, the stiffening area being bonded in compressed form.

For example, a unit serving as a stiffening element and also as an absorption element, with the M and G dimensions indicated above and with the geometry described above but with stiffness which is in itself inadequate, is included in the invention if the necessary stiffness is obtained by being bonded together with other material plies in the area of the stiffening element.

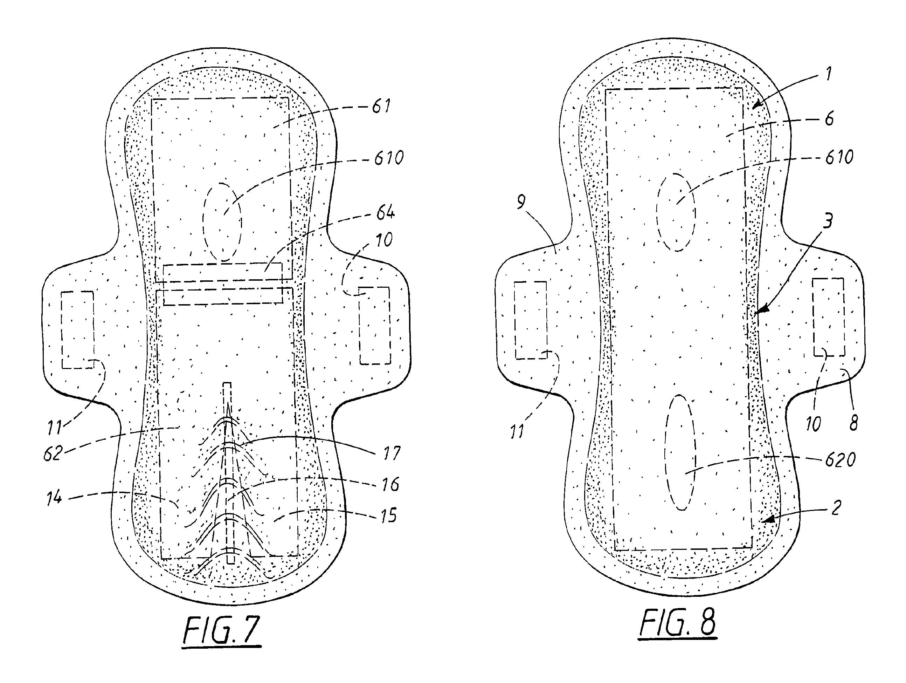

The embodiment shown in Figure 7 differs from the embodiment shown in Figures 4-6 mainly in that the stiffening element 6 consists of a first, front stiffening part element 61 and a second, rear stiffening part element 62 which is separate from the first part element 61 at said transition. The first and the second part element 61 and 62 are thus movable in relation to one another. At the same time, a hinge is formed in the transition area 12 between the two stiffening portions 61 and 62. In the illustrative embodiment shown in Figure 7, the first part element 61 and the second part element 62 are interconnected by means of an elastic film 64, as a result of which said part elements can be moved away from one another counter to the action of the elastic in said elastic film 64.

In the embodiment according to Figure 7, an elastic means 16 is arranged in a pretensioned state in the lorigitudinal direction of the article and centrally along the rear portion 2 of the article. The same reference numbers have been used in Figure 7 as in the embodiment according to Figures 1-6.

The elastic means 16 is arranged centrally in the cutout and extends in the rear portion slightly beyond the ends of the legs 14 and 15 and in the other direction part of the way in over the crotch portion. The elastic means is arranged on the inside or on the outside of the liquidtight outer layer and is connected to the latter and/or other layers forming part of the article. The extent of the elastic means is not critical but can vary somewhat in relation to the illustrative embodiment shown in Figure 3. One purpose of the elastic means 16 is, during use of the article, to draw adjacent material portions together and contribute to curving the article in the upward direction towards the body of the wearer for better contact with the body. Another purpose is also to initiate and form the fold 17 which, during use of the article, is intended to penetrate part of the way into the cleft between the buttocks of the wearer and prevent leakage of bodily fluid backwards along the cleft between the buttocks, which leakage can otherwise occur when the wearer is lying on her back.

In the embodiments shown in Figures 4-7, the stiffening element 6 extends with its leg portions 14, 15 in over the rear portion 2. The outer side edges 18, 19 of the stiffening element 6 on the legs 14, 15 diverge from the crotch portion in over the rear portion in the pressed-together utilization state of the article.

The purpose of the edge sides 18, 19 of the stiffening element diverging in the backward direction on the rear portion 2 during use of the article is that the article, in addition to being anchored firmly at the transition 12 between the front portion and the crotch portion, will also be anchored at the rear in the transition area 20 between the crotch portion 3 and the rear portion 2, as a result of which the article is very stable and well fixed on the wearer during use at the same time as it feels comfortable for the wearer by virtue of its anatomical adaptation in terms of shape, size and geometry. For a good anchoring function, the angle between the longitudinal direction of the article and each outer edge side 18, 19 should not be less than roughly 30°. Furthermore, so as not to feel uncomfortable, the angle should not exceed roughly 60°.

The distance G between the transition areas 12 and 20 is adapted to the crotch length of a wearer and, as mentioned above in connection with the embodiments according to Figures 1-4, this distance G is suitably of the order of 70-120 mm. As mentioned above, the essentially plane area of the crotch of women directly in front of the genitals has a length of in the order of 80-100 mm, that is to say all women are essentially the same size in this plane area. It has been found that a crotch dimension G on the article of in the order of 70-120 mm functions well for most wearers.

It may therefore be suitable to have a range of sizes of the article according to the invention depending on the selection of stiffness and said angles, so that different wearers can find a suitable size with regard to dimensions and angles. This of course applies to all the embodiments of the invention described here but is particularly important when the article is intended to be anchored both at the front and at the rear. The requirement for size adaptation also increases for all the embodiments the stiffer the absorbent element is.

The size of the cutout also influences the height of the fold 17. This height of the fold and the shaping of the back piece 2 also depend on the pretensioning and the extent of the elastic means 16.

Figure 8 shows an embodiment which is slightly modified in relation to the embodiment according to Figure 7. Instead of the cutout 13, an elongate second through-hole 620 is provided, which extends in the longitudinal direction of the article and along the centre line of the article. In this way, the article is during use provided with a fold along the longitudinal direction of the article along said second hole, which fold extends into the cleft between the buttocks of the wearer during use of the article and stabilizes the article in position on the wearer. By virtue of the fact that the stiffening element is continuous behind the second hole, the article according to Figure 8 is significantly more resistant in the rear portion to forces in the lateral direction compared with the embodiment according to Figure 7. In the embodiment according to Figure 7, the legs 14, 15 are of course movable in relation to one another, and the stiffening element is thus more flexible in the rear portion.

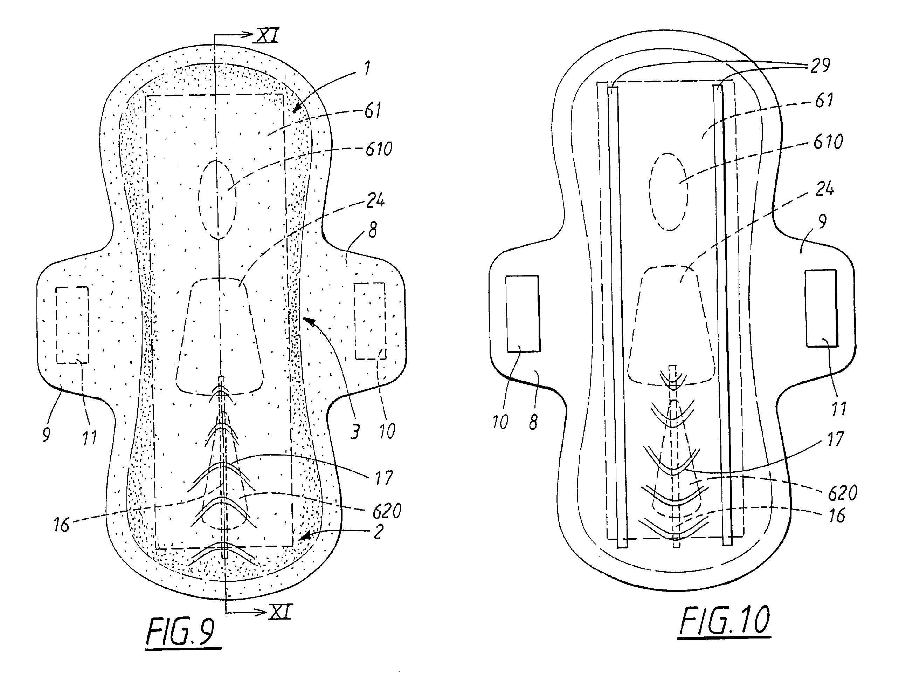

Figures 9-12 show a suitable embodiment of an article according to the invention. This embodiment corresponds in many respects to the embodiments described above, and those parts corresponding to the same parts in the embodiments described above have been provided with the same reference numbers in the drawing.

A way of reducing further the risk of edge leakage caused by the sanitary towel being deformed during use, in addition to the arrangement of the stiffening element 6, is to provide the sanitary towel with a raised portion, what is known as a hump, which raised portion has been designated by reference number 240. The raised portion or hump is intended to make contact with the genitals of the wearer during use of the sanitary towel. Discharged bodily fluid can in this way be caught as soon as it leaves the body of the wearer and be absorbed immediately into the article instead of running out over the surface of the latter.

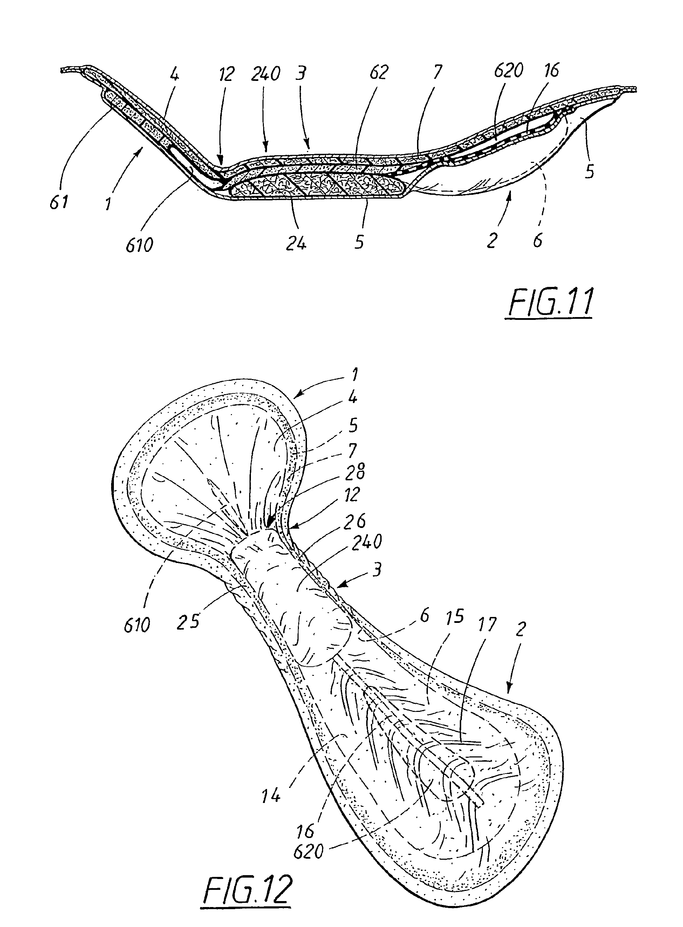

In the embodiment shown in Figures 9-12, the hump is brought about by a hump-forming element 24 which, as can be seen most clearly from Figure 11, is arranged below the stiffening element 6 inside the liquid-impermeable outer layer 5. The positioning of the hump-forming element results in a number of advantages. Admission of bodily fluid is not interfered with by hump material in direct proximity to the genitals of the wearer, but the parts located closest to the genitals of the wearer can be optimized with regard to admission and absorption capacity. The positioning selected for the hump-forming element below the stiffening element 6 in combination with the positioning along the crotch portion of the article also results in the positive effect that the article curves and shapes itself in the desired manner when fitted on the wearer. At the transition 12 between the crotch portion 3 and the front portion, as can be seen from Figure 12, a point of inflexion 27 is formed, in front of which, that is to say in the front portion of the article, the article is concave at least over a portion next to said transition 12. Behind said point of inflexion, that is to say along the crotch portion of the article, the article is, in the area directly in front of the hump-forming element 24, convex, that is to say the stiffening element 6 is curved in this area, upwards in the crotch portion 3, as can be seen most clearly from Figures 11 and 12. In addition to bringing about the raised portion 240 on the front side of the article, the hump-forming element makes it possible to guide the stiffening element in the desired direction of curvature at different points of the extent of the stiffening element.

The hump-forming element 24 consists of, for example, a non-absorbent synthetic wadding which has resilient properties. Such a hump-forming element retains its shape and function even when the material is in a wet state.

The hump-forming element can also consist of a foamed material, for example polyurethane foam or the like.

As the hump-forming material is, in the embodiment shown, located below the absorbent element 6, which also serves as the stiffening element, the hump-forming material can be liquid-absorbing. In such a design, it is suitable to select a material which has larger capillaries than the absorption element has, so that liquid can be transported to the hump-forming material only when the absorption element is saturated with liquid. A hump-forming absorbent fibrous layer which has resilient properties only in the dry state can therefore also be used in such a construction because the material is essentially dry until the absorption element itself is saturated with liquid. The positioning of the hump-forming element 24 below both the stiffening and the absorbent element therefore affords a number of important advantages.

The element forming the raised portion 240 has an elongate shape and extends over the entire crotch portion in the illustrative embodiment shown. The length of the raised portion can vary between roughly 20 mm and 120 mm.

The element 24 forming the raised portion is narrower than the rest of the article in the crotch area. In this way, it is made possible for laterally surrounding portions 25, 26 of the rest of the article to shape themselves around the element 24 forming the raised portion. The material forming the raised portion is suitably at least twice as thick as the surrounding areas 25, 26.

In Figure 11, the article has been shown in curved, three-dimensional form for the sake of clarity. An absorbent article of the type described here is of course always three-dimensional in the conventional sense, that is to say it has length, width and thickness.

In this context, however, the term three-dimensional means that the article must be curved in some way to adapt to the body shape of the wearer.

In this context, the term plane form means that the article is essentially plane. The article shown in Figures 9 and 10 is essentially planiform according to this definition in spite of the fact that the elastic means draws the material layers together in the hole 620 between the legs 14, 15.

Articles in plane form according to Figures 9 and 10 can be packed simply, for example in stacks in a box or bag and yet, when put on, be made to adopt an anatomically adapted three-dimensional shape, as shown in Figures 11 and 12, without any measures whatsoever.

By virtue of its special design with the dimension of the distance M between said muscle tendons in the pressed-together utilization state of the article, the hump-shaped element 24, the action of the hole 620 and of the elastic means 16 and the stiffness and geometric shape of the stiffening element 6, the article is anatomically adapted and predestined to adopt during handling a three-dimensional shape according to Figures 11 and 12 adapted to the body shape of the wearer.

In the illustrative embodiment shown, the stiffening and also absorbent element 6 has the same stiffness properties over its entire extent. As a result, uncontrolled creases, which could give rise to uncontrolled and unintentional liquid flow, do not normally arise over the extent of the stiffening element. At the transition 12 between the crotch portion 3 and the front portion 1, curvature is initiated because the article as a whole changes its flexural resistance here, on the one hand on account of the hump-forming element having its end directly in front of this transition and on the other hand because the stiffening element has the least material here and is narrowest here in the utilization state with a dimension M adapted to the distance between said muscle tendons on the wearer. At this transition 12, a point of inflexion 27 is formed, in front of which the article is concave and bowl-shaped, whereas it adopts a convex shape behind this point of inflexion 27. In the embodiment according to Figure 12, the hump-forming element is rounded at the front along a line 28. In this way, the stiffening element is caused by this rounded line to adopt an evenly rounded bowl shape in the front portion, as can be seen from Figure 12.

In the transition area 20 between the crotch portion 3 and the rear portion 2 as well, the hump-forming element 24, which in the embodiment shown extends as far as said transition area 20, is rounded at its rear end. As a result, no undesirable creases arise, but the transition between the convex crotch portion and the two side portions of the rear portion 2 sloping downwards around the fold 17 formed by the elastic means 16 is even and smooth without undesirable creases.

The elongate hole 620 in the rear portion 2 of the article gives rise to a fold in the longitudinal direction of the hole, which fold extends into the cleft between the buttocks of the wearer during use of the article and stabilizes the article in position in the lateral direction. As the width of the elongate hole increases continuously in the backward direction, the height of the fold formed will also increase continuously in the backward direction.

The raised portion 240 formed by the hump-forming element 24 also has the advantage that the fold extending into the cleft between the buttocks of the wearer does not extend in too abruptly or too far and give rise to chafing. In this respect also, the hump provides a soft transition in the transition area between the crotch portion and the rear portion.

In all the embodiments described above, it is suitable for the article to be provided with a pressure-sensitive adhesive on the outside of its liquid-impermeable outer layer 5. This has been indicated in Figure 10 by adhesive strands 29 which, before use of the article, are covered in a conventional manner by a cover strip 30 treated with release agent. Although the article according to the invention is anatomically adapted, it is suitable, for reliable secure positioning, to have a pressure-sensitive adhesive on the liquid-impermeable outside of the article for interaction with the briefs of the wearer, which contributes to keeping the article in the intended position on the wearer.

The selection of suitable attachment, that is to say whether and to what extent pressure-sensitive adhesive for attachment to the briefs of the wearer is to be used, is guided by the selection of the stiffness of the stiffening element included.

According to one embodiment (not shown in the drawing), the article can be attached to or interact with the body of the wearer by means of adhesive or friction coating. The friction means or adhesive can be the only means of attachment, but it can also be used in combination with pressure-sensitive adhesive intended for attachment to the briefs of the wearer.

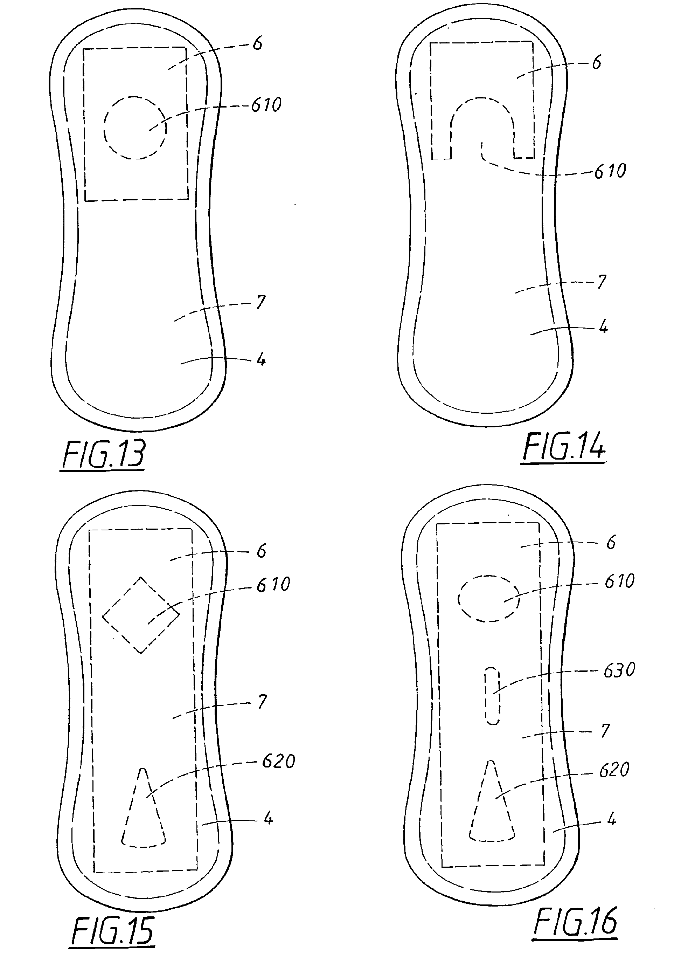

Figures 13-16 show further examples of the stiffening element for an absorbent article according to the invention. In the embodiments according to Figures 13-16, components corresponding to similar components in illustrative embodiments described above have been provided with the same reference numbers.

In the embodiment according to Figure 13, the stiffening element 6 is arranged in the front portion of the article and extends part of the way in over the crotch portion of the article. The stiffening element 6 is provided with a circular through-hole 610, the widest portion of which is located directly in front of the transition 12, that is to say the area where the article should during use be anchored firmly in the pressed-together state between said muscle tendons directly in front of the groin of the wearer.

The embodiment according to Figure 14 differs from the embodiment shown in Figure 13 in that the hole has been replaced by a U-shaped cutout in that portion of the stiffening element 6 facing the crotch portion of the article. The width of the cutout is at a maximum in the transition area 12, where maximum pressing-together of the stiffening element is desired for its anchoring between said muscle tendons during use of the article.

In the embodiment according to Figure 15, the stiffening element 6 extends over the front portion 1, the crotch portion 3 and the rear portion 2 of the article. In the front portion, the stiffening element is provided with a triangular through-hole 610 which is widest in the transition area 12 where the article is intended to be anchored between said muscle tendons during use of the article. By means of the triangular hole, continuously decreasing curvature of the stiffening element is obtained from said transition 12 in the direction towards the front end of the article. In the rear portion 2 of the article, as in the embodiment according to Figures 9-12, a longitudinal hole 620 is arranged. This has the same function as described in the embodiment according to Figures 9-12 and, during use of the article, gives rise to a fold, the height of which increases in the backward direction. During use of the article, the fold stabilizes it in the lateral direction as described above.

Figure 16 shows an article, the stiffening element 6 of which, as in the embodiment according to Figure 15, extends over the front portion 1, the crotch portion 3 and the rear portion 2 of the article. In this case, the stiffening element has an elliptical hole 610 directly in front of the transition 12, which hole is intended, during use of the article, to make possible squeezing-together of the stiffening element at the transition 12 to bring about a narrowing in this area to a width M which corresponds to the distance between said muscle tendons on the wearer. A longitudinal hole 630 is arranged in the crotch area of the article. The purpose of the hole 630 is to provide the stiffening element with resilient properties in the crotch area for optimum adaptation of the width of the stiffening element in the crotch area to the body shape of the wearer in this area. The hole 620 has the same function as the corresponding hole in the embodiment according to Figure 15 and is therefore not described further.

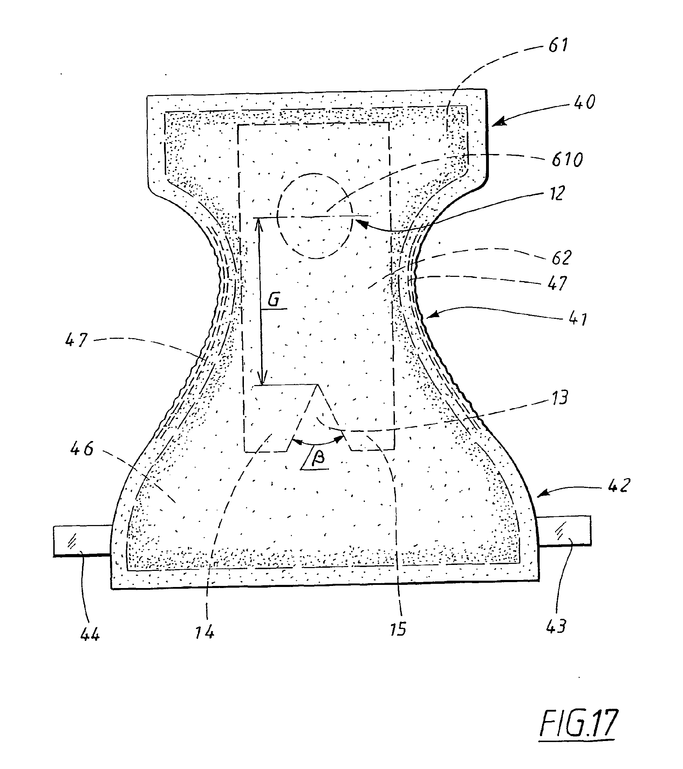

Figure 17 shows an embodiment in the form of a nappy. This has a front portion 40, a crotch portion 41 and a rear portion 42. Outwardly, the embodiment shown of an article in the form of a nappy according to the present invention is of conventional design. When the nappy is put on, the front portion 40 and the rear portion 42 are intended to be fitted around the waist of the wearer and to be closed in the fitted position by means of tape flaps 43, 44. In Figure 17, the nappy is shown diagrammatically in plane form from the inside and is provided with a covering in the form of a liquid-permeable inner layer 45, suitably made of non-woven fabric, and an outer layer made of thin plastic film (not shown), suitably made of polyethylene. Inside the inner layer, an essentially hourglass-shaped absorption layer 46 is indicated, which is thin and very flexible. In the crotch portion, leg elastic 47, 48, which is intended to fit tightly around the thighs of the wearer during use of the nappy, has been arranged along the edge portion.

Figure 17 shows diagrammatically a stiffening and also absorbent element 6 of the same type as described in the illustrative embodiments described above. In Figure 17, components corresponding to similar parts in illustrative embodiments described above have been provided with the same reference numbers. The stiffening and absorbent element is anatomically adapted in the same way as in illustrative embodiments described above with a dimension M in the utilization state adapted to the distance between said muscle tendons directly in front of the groins and with a crotch length G adapted to the crotch length of the wearer and with angles and geometry in general adapted in the same way as described above during use of the article in the pressed-together state. The stiffening element has a rectangular shape with a through-hole 610 in the front portion and at the transition 12 between the front portion 40 and the crotch portion 41, and with a cutout 13 in the rear portion 42 of the article.

As mentioned above, a person has essentially the same dimension M throughout his or her life. The length of the crotch is also essentially of the same order of size in infants and adults. Nappies according to Figure 17 therefore function in principle for both children and adults if the article as a whole is adapted in terms of size.

A nappy according to the invention of the type shown in Figure 17 has a superior fit compared with conventional nappies. The presence of the stiffening element means that, when the nappy is put on, it is guided into the correct position on the wearer and that it remains in this position during use of the article.

In all the illustrative embodiments described above, the width of the stiffening and also absorbent element 6 increases continuously in the pressed-together utilization state from the transition 12 between the front portion 1 and the crotch portion 3 to the transition area 20 between the crotch portion 3 and the rear portion. One reason for this is that the available space between the legs of the wearer is very limited, and it is important to make optimum use of the width of this area. The width can increase by of the order of 1.5 times between the transition 12 and the transition area 20 without this feeling uncomfortable for the wearer. Another reason is that the article lies more stably on the wearer when the stiffening element is made as wide as possible along the crotch portion.

The invention is not limited to the illustrative embodiments described above, but a large number of modifications are possible within the scope of the patent claims below.

The illustrative embodiments described above have shown articles with one first cutout 610. Within the scope of the invention, the article can also be provided with a number of first cutouts 610 arranged in the lateral direction at a distance from one another. If a number of first cutouts are present, these are suitably arranged symmetrically in relation to a longitudinal centre line.

For example, anatomically shaped stiffening and absorbent elements of the type described above can be arranged in what are known as pant nappies, that is to say where the nappy is integrated into disposable pants.

It has been stated above that the stiffening absorbent element can be made from different materials and from laminates made of one or more material(s). The stiffening absorbent element can also be made from more than one layer and with the extent of the individual layers being different, in which way it is possible for different areas of the stiffening element to have different stiffness.

As mentioned above, the stiffening element can consist of all the material layers and bonding agents included. Different stiffness in different areas of the stiffening element can therefore also be obtained by varying the degree of connection in different areas, for example different quantities of adhesive in different areas and even the absence of adhesive or other bonding agent in different areas between or in individual layers.

The elastic means 16, which is arranged along the cutout 13, has been indicated in the illustrative embodiments described above as having been arranged in a pretensioned state. However, in the manufacture of absorbent articles such as sanitary towels, nappies and the like, it is known to arrange a heat-sensitive elastic means in an untensioned state and to tension the elastic by heat treatment. This suitably takes place when the articles are packed.

In the illustrative embodiments described above, the stiffening element 6 and the stiffening part elements 61 and 62 have a rectangular basic shape. The stiffening element 6 and the part elements 61 and 62 can have a different basic shape which is better suited to the body shape of the wearer. The stiffening element can be, for example, hourglass-shaped or provided with legs diverging in the rear portion.

In the illustrative embodiments described above relating to articles for arrangement inside the crotch portion of briefs, the article is in the majority of the illustrative embodiments provided with permanently arranged wings for attachment of the article to the briefs with the wings folded around the edge portion of the briefs and attached on the outside of the crotch portion. The wings can consist of separate elements which are attached to the rest of the article in connection with the article being put on. The separate wings can be arranged detachably on the rest of the article during manufacture of the article, as a result of which a wearer who does not want to have wings on the article can remove these in connection with putting the article on.

The illustrative embodiments described above which do not have wings can be provided with separate wings either during manufacture or when the article is put on.

Within the scope of the patent claims below, the article according to the invention can be made from one piece of material, for example a moulded foamed material with open pores. These pores can be closed on the rear side of the article and along the entire outer edge contour to form a liquidtight layer. In this embodiment, stiffening areas are produced by the foamed material being compressed and bonded in the compressed state. To increase the absorption capacity, a moulded shaped body made of foamed material can contain superabsorbent materials in the form of particles or fibres bonded in the foam structure.

## Claims

1. Absorbent article, such as a sanitary towel, a panty liner, an incontinence pad, a nappy or the like, which article has a longitudinal direction and a transverse direction, a front portion (1), a rear portion (2), a crotch portion (3) located between the rear portion and the front portion, an absorbent element and a liquidtight layer (5), and also a stiffening element (6) which is intended to contribute to the three-dimensional shape of the article during its use, **characterized in that** the stiffening element (6) is in a plane state before use of the article, **in that** the stiffening element (6) is provided with at least one first cutout (610) which is arranged so as to make possible when the article is in use pressing-together of the stiffening element in the transverse direction at a transition (12) between the front portion and the crotch portion, **in that** the shape of the stiffening element (6) and the shape of the first cutout (610) are adapted so that the stiffening element, after pressing-together and positioning on the wearer, has a width (M) at said transition (12) which is adapted to the distance between the muscle tendons of the wearer on both sides of the crotch of the wearer in the groin of the latter and which width (M) is of the order of 15-35 mm, **in that** the stiffening element (6) is arranged so that its side edges in the front portion (1) of the article, in said pressed-together utilization state, can diverge in the direction from the crotch portion (3) at least part of the way in over the front portion (1), and can form an acute angle (α) with a line in the longitudinal direction of the article, **in that**, in the plane state before use, the stiffening element (6) has a width at the transition (12) between the front portion (1) and the crotch portion (3) which exceeds 45 mm, and **in that** the stiffening element is absorbent.

2. Article according to Claim 1, **characterized in that** the stiffening element (6) extends in the longitudinal direction of the article over at least part of the front portion (1), and **in that** the first cutout (610) is symmetrical in relation to a centre line along the longitudinal direction of the article and extends over at least a portion of the front portion (1).

3. Article according to any one of the preceding claims, **characterized in that** the stiffening element (6) at the same time constitutes the absorbent element, and **in that**, during absorption in the pressed-together utilization state, it is adapted so as to swell while on the whole retaining its geometry in the transverse direction and longitudinal direction of the article.

4. Article according to any one of the preceding claims, **characterized in that**, in the pressed-together utilization state, said width (M) of the stiffening element (6) at the transition between the crotch portion and the front portion is of the order of 20-35 mm.

5. Article according to any one of the preceding claims, **characterized in that**, in the pressed-together utilization state, said width (M) of the stiffening element (6) at the transition between the crotch portion (3) and the front portion (1) is of the order of 25-30 mm.

6. Article according to any one of the preceding claims, **characterized in that** the stiffening element (6) has a stiffness in the dry state of in the order of 1-15 N measured according to ASTM D 4032-82.

7. Article according to any one of the preceding claims, **characterized in that** the stiffening element (6) consists of a dry-formed fibre mat with a density between 0.15 and 0.75 g/cm³ and a weight per unit area of in the order of 100-400 g/m².

8. Article according to Claim 7, **characterized in that** the dry-formed fibre mat is, after compression, mechanically softened to the desired stiffness.

9. Article according to Claim 7 or 8, **characterized in that** the dry-formed fibre mat is provided with the desired stiffness and the desired extensibility by virtue of the degree of compression selected and the compression pattern selected.

10. Article according to any one of the preceding claims, **characterized in that** the side edges of the stiffening element (6), which, in the pressed-together utilization state of the article, diverge at least part of the way from the crotch portion (3) in over the front portion (1) of the article, are arranged so as in said state to form an angle (α) between a line in the longitudinal direction of the article and each of said side edges of in the order of 35-55°, preferably in the order of 45°.

11. Article according to any one of the preceding claims, **characterized in that** the stiffening element (6) also extends part of the way in over the rear portion (2) of the article, and **in that** the stiffening element is arranged so that its side edges (18, 19), in the pressed-together utilization state of the article, diverge in the direction from the crotch portion (3) at least part of the way from the crotch portion in over the rear portion of the article.

12. Article according to Claim 11, **characterized in that** the stiffening element (6) has in the rear portion (2) a second cutout (13) extending from its end edge in the direction towards the crotch portion (3), as a result of which the article is during use provided with a fold (17) along the longitudinal direction of the article in said second cutout, which fold extends into the cleft between the buttocks of the wearer during use of the article.

13. Article according to Claim 12, **characterized in that** said second cutout (13) is wedge-shaped and located symmetrically and forms an angle (β) of between 10° and 120°, preferably between 15° and 40°, at its end facing the crotch portion.

14. Article according to Claim 11, **characterized in that** the stiffening element (6) has in the rear portion (2) an elongate second through-hole (620) which extends in the longitudinal direction of the article and along the centre line of the article, as a result of which the article is during use provided with a fold along the longitudinal direction of the article along said second hole, which fold extends into the cleft between the buttocks of the wearer during use of the article and in this way stabilizes the article in position on the wearer.

15. Article according to any one of the preceding claims, **characterized in that** the stiffening element (6) has a stiffness of at least 1.0 N, and **in that** the stiffening element is designed with essentially the same stiffness over the entire extent of the stiffening element.

16. Article according to any one of the preceding claims, **characterized in that** the stiffening element (6), seen in the longitudinal direction of the article, comprises a first, front stiffening part element (61) which, in the pressed-together utilization state, has said width (M) at the transition (12) between the front portion and the crotch portion, and at least one further, second stiffening part element (62) which is separate from the first part element (61) at said transition (12), as a result of which the first and the second part element are movable in relation to one another.

17. Article according to Claim 16, **characterized in that** the second stiffening part element (62) also extends part of the way in over the rear portion (2) of the article, and **in that**, in the pressed-together utilization state of the article, the side edges (18, 19) of the second stiffening element are arranged so as, in the direction from the crotch portion (3), to diverge at least part of the way from the crotch portion in over the rear portion (2) of the article.

18. Article according to Claim 16 or 17, **characterized in that** said second cutout (13) is arranged in the second stiffening part element (62).

19. Article according to any one of the preceding claims, **characterized in that** the stiffening element (6) extends over said transition (12), and at least part of the way over the front portion (1) and also at least part of the way over the crotch portion, and **in that** the first cutout (610) consists of at least one hole which is widest at least directly in front of the transition (12) and is circular or oblong.

20. Article according to any one of the preceding claims, **characterized in that** the stiffening element (6) has, in the plane state before use, the same width along its length with the exception of the end portions which suitably are rounded.

21. Article according to Claim 16, **characterized in that** the first cutout (610) consists of a cutout open towards the transition (12).

22. Article according to any one of Claims 19-21, **characterized in that** the first cutout (610) is in its entirety located in the front portion (1) and is essentially diamond-shaped.

23. Article according to any one of the preceding claims, **characterized in that** at least one elastic means (16) is arranged in the longitudinal direction of the article and centrally along the rear portion of the article and along at least part thereof from the crotch portion, which elastic means is intended, along its length, to draw adjacent material portions together and curve the article upwards for better contact with the body of the wearer.

24. Article according to any one of the preceding claims, **characterized in that** the stiffening element (6) serves as an absorption means and has very great liquid-spreading capacity for spreading bodily fluid received in the relatively narrow crotch area (3) bounded by the anatomy of the wearer directly in front of the genitals of the wearer over the absorbent portions of the whole article, and **in that** the stiffening element is designed with great swelling capacity in the depth direction and attendant great absorption capacity.

25. Article according to Claim 24, **characterized in that** the stiffening element (6) also serves as an absorption element and is essentially homogeneous over its entire extent with regard to thickness, stiffness, spreading capacity and absorption capacity, as a result of which the stiffening layer and thus also the absorption element curve evenly during use without forming local irregularities which may give rise to undesirable spreading of liquid.

26. Article according to any one of the preceding claims, **characterized in that** the stiffening element (6) also constitutes the absorbent element, and **in that** the shape thereof and of the first cutout (610) is adapted so that, when the stiffening element (6) is pressed together between the muscle tendons of the wearer in the groins during use of the article, the width of the stiffenin element behind said transition area (12) increases continuously in the crotch portion in the backward direction towards the rear portion (2) for the purpose of optimally utilizing available width space in this area with regard to maximum absorption.

27. Article according to any one of the preceding claims, **characterized in that** said stiffening element (6) consists of at least one compressed area of a continuous material body made in one piece.

28. Article according to any one of the preceding claims, **characterized in that** a hump-forming element (24) made of a resilient material is arranged under the stiffening element over at least part of the crotch portion (3), which hump-forming element is arranged so as to bring about a raised portion (240) on the side which is intended to be fitted against the wearer, the raised portion being arranged so as to come to lie directly in front of the genitals of the wearer after fitting of the article on the wearer.

29. Article according to Claim 28, **characterized in that** the raised portion (240) is elongate in the longitudinal direction of the article and has a length of between 20 mm and 120 mm.

30. Article according to Claim 28 or 29, **characterized in that** the raised portion (240) is narrower than the rest of the article in the crotch area, and **in that** the raised portion is thicker than surrounding areas.

## Patentansprüche

1. Saugfähiger Artikel, wie beispielsweise eine Binde, eine Slipeinlage, eine Inkontinenzeinlage, eine Windel oder dergleichen, welcher Artikel eine Längsrichtung und eine Querrichtung, einen Vorderteil (1), einen Hinterteil (2), einen Schrittteil (3), der sich zwischen dem Hinterteil und dem Vorderteil befindet, ein saugfähiges Element und eine flüssigkeitsdichte Schicht (5) und ebenfalls ein Versteifungselement (6) aufweist, welches eingerichtet ist, um zur dreidimensionalen Form des Artikels während seiner Verwendung beizutragen, **dadurch gekennzeichnet, dass** sich das Versteifungselement (6) vor der Verwendung des Artikels in einem ebenen Zustand befindet, dass das Versteifungselement (6) mit zumindest einem ersten Ausschnitt (610) versehen ist, welcher dazu ausgebildet ist, bei der Verwendung des Artikels das Zusammendrücken des Versteifungselements in der Querrichtung an einem Übergang (12) zwischen dem Vorderteil und dem Schrittteil zu ermöglichen, dass die Form des Versteifungselements (6) und die Form des ersten Ausschnitts (610) so ausgelegt sind, dass das Versteifungselement nach dem Zusammendrücken und Positionieren am Benutzer eine Breite (M) am Übergang (12) aufweist, welche dem Abstand zwischen den Muskelsehnen des Benutzers auf beiden Seiten des Schritts des Benutzers in der Leiste des Letzteren angepasst ist, und welche Breite (M) in der Größenordnung von 15-35 mm ist, dass das Versteifungselement (6) so angeordnet ist, dass seine Seitenkanten im Vorderteil (1) des Artikels, im zusammengedrückten Verwendungszustand, in der Richtung vom Schrittteil (3) zumindest einen Teil der Strecke über den Vorderteil (1) abzweigen können und einen spitzen Winkel (α) mit einer Linie in der Längsrichtung des Artikels bilden können, dass das Versteifungselement (6) im ebenen Zustand vor der Verwendung eine Breite am Übergang (12) zwischen dem Vorderteil (1) und dem Schrittteil (3) aufweist, welche 45 mm übersteigt, und dass das Versteifungselement saugfähig ist.

2. Artikel nach Anspruch 1, **dadurch gekennzeichnet, dass** sich das Versteifungselement (6) in der Längsrichtung des Artikels über zumindest einen Teil des Vorderteils (1) erstreckt, und dass der erste Ausschnitt (610) im Verhältnis zu einer Mittellinie entlang der Längsrichtung des Artikels symmetrisch ist und sich über zumindest einen Teil des Vorderteils (1) erstreckt.

3. Artikel nach irgendeinem der vorhergehenden Ansprüche,
**dadurch gekennzeichnet dass** das Versteifungselement (6) gleichzeitig das saugfähige Element bildet, und dass es während der Absorption im zusammengedrückten Verwendungszustand zum Anschwellen ausgelegt ist, während es insgesamt seine Geometrie in der Querrichtung und Längsrichtung des Artikels beibehält.

4. Artikel nach irgendeinem der vorhergehenden Ansprüche,
**dadurch gekennzeichnet, dass** im zusammengedrückten Verwendungszustand die Breite (M) des Versteifungselements (6) am Übergang zwischen dem Schrittteil und dem Vorderteil in der Größenordnung von 20-35 mm liegt.

5. Artikel nach irgendeinem der vorhergehenden Ansprüche,
**dadurch gekennzeichnet, dass** im zusammengedrückten Verwendungszustand die Breite (M) des Versteifungselements (6) am Übergang zwischen dem Schrittteil (3) und dem Vorderteil (1) in der Größenordnung von 25-30 mm liegt.

6. Artikel nach irgendeinem der vorhergehenden Ansprüche,
**dadurch gekennzeichnet, dass** das Versteifungselement (6) eine Steifheit im Trockenzustand der Größenordnung von 1-15 N, gemessen nach ASTM D 4032-82, aufweist.

7. Artikel nach irgendeinem der vorhergehenden Ansprüche,
**dadurch gekennzeichnet, dass** das Versteifungselement (6) aus einer trocken-gebildeten Fasermatte mit einer Dichte zwischen 0,15 und 0,75 g/cm³ und einem Flächengewicht in der Größenordnung von 100-400 g/m² besteht.

8. Artikel nach Anspruch 7, **dadurch gekennzeichnet, dass** die trockengebildete Fasermatte nach der Kompression auf die gewünschte Steifheit mechanisch aufgeweicht ist.

9. Artikel nach Anspruch 7 oder 8, **dadurch gekennzeichnet, dass** die trocken-gebildete Fasermatte mit der gewünschten Steifheit und der gewünschten Dehnbarkeit durch den ausgewählten Kompressionsgrad und das ausgewählte Kompressionsmuster versehen ist.

10. Artikel nach irgendeinem der vorhergehenden Ansprüche,
**dadurch gekennzeichnet, dass** die Seitenkanten des Versteifungselements (6), die im zusammengedrückten Verwendungszustand des Artikels zumindest einen Teil der Strecke vom Schrittteil (3) über den Vorderteil (1) des Artikels abzweigen, so angeordnet sind, dass sie in diesem Zustand einen Winkel (α) zwischen einer Linie in der Längsrichtung des Artikels und jeder der Seitenkanten in der Größenordnung von 35-55°, bevorzugt in der Größenordnung von 45°, bilden.

11. Artikel nach irgendeinem der vorhergehenden Ansprüche,
**dadurch gekennzeichnet, dass** sich das Versteifungselement (6) ebenfalls einen Teil der Strecke über den Hinterteil (2) des Artikels erstreckt, und dass das Versteifungselement so angeordnet ist, dass seine Seitenkanten (18, 19) im zusammengedrückten Verwendungszustand des Artikels in der Richtung vom Schrittteil (3) zumindest einen Teil der Strecke vom Schrittteil über den Hinterteil des Artikels abzweigen.

12. Artikel nach Anspruch 11, **dadurch gekennzeichnet, dass** das Versteifungselement (6) im Hinterteil (2) einen zweiten Ausschnitt (13) aufweist, der sich von seiner Endkante in Richtung des Schrittteils (3) erstreckt, wodurch der Artikel während der Verwendung mit einer Falte (17) entlang der Längsrichtung des Artikels im zweiten Ausschnitt versehen ist, welche Falte sich in die Spalte zwischen den Gesäßhälften des Benutzers während der Verwendung des Artikels hineinstreckt.

13. Artikel nach Anspruch 12, **dadurch gekennzeichnet, dass** der zweite Ausschnitt (13) keilförmig und symmetrisch angeordnet ist und einen Winkel (ß) von zwischen 10° und 120°, bevorzugt zwischen 15° und 40°, an seinem dem Schrittteil zugewandten Ende bildet.

14. Artikel nach Anspruch 11, **dadurch gekennzeichnet, dass** das Versteifungselement (6) im Hinterteil (2) ein langgestrecktes zweites Durchgangsloch (620) aufweist, das sich in der Längsrichtung des Artikels und entlang der Mittellinie des Artikels erstreckt, wodurch der Artikel während der Verwendung mit einer Falte entlang der Längsrichtung des Artikels entlang des zweiten Lochs versehen ist, welche Falte sich in die Gesäßhälften des Benutzers während der Verwendung des Artikels hineinstreckt und auf diese Weise den am Benutzer positionierten Artikel stabilisiert.

15. Artikel nach irgendeinem der vorhergehenden Ansprüche,
**dadurch gekennzeichnet, dass** das Versteifungselement (6) eine Steifheit von zumindest 1,0 N aufweist, und dass das Versteifungselement mit im Wesentlichen der gleichen Steifheit über die Gesamtausdehnung des Versteifungselements ausgebildet ist.

16. Artikel nach irgendeinem der vorhergehenden Ansprüche,
**dadurch gekennzeichnet, dass** das Versteifungselement (6), in der Längsrichtung des Artikels gesehen, ein erstes, vorderes Versteifungsteilelement (61), welches im zusammengedrückten Verwendungszustand die Breite (M) am Übergang (12) zwischen dem Vorderteil und dem Schrittteil aufweist, und zumindest ein zusätzliches, zweites Versteifungsteilelement (62) umfasst, welches vom ersten Teilelement (61) am Übergang (12) getrennt ist, wodurch das erste und das zweite Teilelement im Verhältnis zu einander beweglich sind.

17. Artikel nach Anspruch 16, **dadurch gekennzeichnet, dass** sich das zweite Versteifungsteilelement (62) ebenfalls einen Teil der Strecke über den Hinterteil (2) des Artikels erstreckt, und dass die Seitenkanten (18, 19) des zweiten Versteifungselements im zusammengedrückten Verwendungszustand des Artikels so angeordnet sind, dass sie in der Richtung vom Schrittteil (3) zumindest einen Teil der Strecke vom Schrittteil über den Hinterteil (2) des Artikels abzweigen.

18. Artikel nach Anspruch 16 oder 17, **dadurch gekennzeichnet, dass** der zweite Ausschnitt (13) im zweiten Versteifungsteilelement (62) angeordnet ist.

19. Artikel nach irgendeinem der vorhergehenden Ansprüche,
**dadurch gekennzeichnet, dass** sich das Versteifungselement (6) über den Übergang (12) und zumindest einen Teil der Strecke über den Vorderteil (1) und ebenfalls zumindest einen Teil der Strecke über den Schrittteil erstreckt, und dass der erste Ausschnitt (610) aus zumindest einem Loch besteht, das zumindest direkt vor dem Übergang (12) am breitesten ist und kreisförmig oder länglich ist.

20. Artikel nach irgendeinem der vorhergehenden Ansprüche,
**dadurch gekennzeichnet, dass** das Versteifungselement (6) im ebenen Zustand vor der Verwendung die gleiche Breite entlang seiner Länge aufweist, mit Ausnahme von den Endteilen, die in angemessener Weise gerundet sind.

21. Artikel nach Anspruch 16, **dadurch gekennzeichnet, dass** der erste Ausschnitt (610) aus einem Ausschnitt besteht, der in Richtung des Übergangs (12) offen ist.

22. Artikel nach irgendeinem der Ansprüche 19-21,
**dadurch gekennzeichnet, dass** der erste Ausschnitt (610) in seiner Gesamtheit im Vorderteil (1) angeordnet ist und im Wesentlichen rautenförmig ist.

23. Artikel nach irgendeinem der vorhergehenden Ansprüche,
**dadurch gekennzeichnet, dass** zumindest ein elastisches Mittel (16) in der Längsrichtung des Artikels und mittig entlang des Hinterteils des Artikels und entlang zumindest eines Teils davon aus dem Schrittteil angeordnet ist, wobei das elastische Mittel dafür vorgesehen ist, die benachbarten Materialteile zuzuziehen, und den Artikel zum besseren Kontakt mit dem Körper des Benutzers nach oben zu biegen.

24. Artikel nach irgendeinem der vorhergehenden Ansprüche,
**dadurch gekennzeichnet, dass** das Versteifungselement (6) als ein Absorptionsmittel dient und eine sehr große Flüssigkeitsausbreitungs-Fähighkeit zur Ausbreitung von Körperflüssigkeit aufweist, die im relativ schmalen Schrittbereich (3) aufgenommen wird, begrenzt durch die Anatomie des Benutzers direkt vor den Genitalien des Benutzers über den saugfähigen Teilen des ganzen Artikels, und dass das Versteifungselement mit großer Fähigkeit zum Anschwellen in der Tiefenrichtung und dazugehöriger großer Absorptionsfähigkeit ausgebildet ist.

25. Artikel nach Anspruch 24, **dadurch gekennzeichnet, dass** das Versteifungselement (6) ebenfalls als ein Absorptionselement dient und bezüglich Dicke, Steifheit, Ausbreitungsfähighkeit und Absorptionsfähigkeit im Wesentlichen über seine ganze Ausdehnung homogen ist, wodurch sich die Versteifungsschicht und damit auch das Absorptionselement während der Verwendung gleichmäßig krümmen, ohne dass lokale Unregelmäßigkeiten gebildet werden, welche zur unerwünschten Ausbreitung von Flüssigkeit führen können.

26. Artikel nach irgendeinem der vorhergehenden Ansprüche,
**dadurch gekennzeichnet, dass** das Versteifungselement (6) ebenfalls das saugfähige Element bildet, und dass die Form davon und des ersten Ausschnitts (610) so ausgelegt ist, dass sich die Breite des Versteifungselements hinter dem Übergangsbereich (12), wenn das Versteifungselement (6) zwischen den Muskelsehnen des Benutzers in den Leisten während der Verwendung des Artikels zusammengedrückt wird, zum Zwecke der optimalen Verwendung des zur Verfügung stehenden Breitraums in diesem Bereich bezüglich der maximalen Absorption im Schrittteil in der Rückwärtsrichtung in Richtung des Hinterteils (2) ständig vergrößert.

27. Artikel nach irgendeinem der vorhergehenden Ansprüche,
**dadurch gekennzeichnet, dass** das Versteifungselement (6) aus zumindest einem komprimierten Bereich eines einstückig hergestellten kontinuierlichen Materialkörpers besteht.

28. Artikel nach irgendeinem der vorhergehenden Ansprüche,
**dadurch gekennzeichnet, dass** das aus einem nachgiebigen Material hergestellte Buckelbildungselement (24) unter dem Versteifungselement über zumindest einen Teil des Schrittteils (3) angeordnet ist, wobei das Buckelbildungselement so angeordnet ist, um einen erhöhten Teil (240) auf der Seite vorzusehen, der eingerichtet ist, um gegen den Benutzer angebracht zu werden, wobei der erhöhte Teil so angeordnet wird, dass er direkt vor den Genitalien des Benutzers anliegt, nachdem der Artikel am Benutzer angebracht worden ist.

29. Artikel nach Anspruch 28, **dadurch gekennzeichnet, dass** der erhöhte Teil (240) in der Längsrichtung des Artikels langgestreckt ist und eine Länge von zwischen 20 mm und 120 mm aufweist.

30. Artikel nach Anspruch 28 oder 29, **dadurch gekennzeichnet, dass** der erhöhte Teil (240) schmaler als der Rest des Artikels im Schrittbereich ist, und dass der erhöhte Teil dicker als umgebende Bereiche ist.

## Revendications

1. Article absorbant, tel qu'une serviette hygiénique, un protège-slip, une protection contre l'incontinence, une couche-culotte ou similaire, ledit article présentant une direction longitudinale et une direction transversale, une partie avant (1), une partie arrière (2), une partie d'entre-jambes (3) située entre la partie arrière et la partie avant, un élément absorbant et une couche imperméable aux liquides (5), et aussi un élément de raidissement (6) qui est destiné à contribuer à la forme tridimensionnelle de l'article pendant son utilisation, **caractérisé en ce que** l'élément de raidissement (6) est dans un état plan avant l'utilisation de l'article, **en ce que** l'élément de raidissement (6) est pourvu d'au moins une première découpe (610) qui est agencée de manière à permettre, lorsque l'article est utilisé, une compression de l'élément de raidissement dans la direction transversale à une transition (12) entre la partie avant et la partie d'entre-jambes, **en ce que** la forme de l'élément de raidissement (6) et la forme de la première découpe (610) sont adaptées si bien que le l'élément de raidissement, après la compression et le positionnement sur le porteur, présente une largeur (M) au niveau de ladite transition (12) qui est adaptée à la distance entre les tendons musculaires du porteur de chaque côté de l'entre-jambes du porteur dans l'aine de ce dernier et ladite largeur (M) est comprise entre 15 et 35 mm, et **en ce que** l'élément de raidissement (6) est disposé si bien que ses bords latéraux dans la partie avant (1) de l'article, dans ledit état d'utilisation comprimé, peuvent diverger en direction de la partie d'entre-jambes (3) au moins une portion du trajet sur la partie avant (1), et peuvent former un angle aigu (α) avec une ligne dans la direction longitudinale de l'article, **en ce que**, dans l'état plan avant l'utilisation, l'élément de raidissement (6) présente une largeur au niveau de la transition (12) entre la partie avant (1) et la partie d'entre-jambes (3) qui dépasse 45 mm, et **en ce que** l'élément de raidissement est absorbent.

2. Article selon la revendication 1, **caractérisé en ce que** l'élément de raidissement (6) s'étend dans la direction longitudinale de l'article sur au moins une portion de la partie avant (1), et **en ce que** la première découpe (610) est symétrique par rapport à une ligne centrale le long de la direction longitudinale de l'article et s'étend sur au moins une portion de la partie avant (1).

3. Article selon l'une quelconque des revendications précédentes, **caractérisé en ce que** l'élément de raidissement (6) dans le même temps constitue l'élément absorbant, et **en ce que**, lors de l'absorption dans l'état d'utilisation comprimé, il est adapté de manière à gonfler dans l'ensemble en conservant sa géométrie dans la direction transversale et la direction longitudinale de l'article.

4. Article selon l'une quelconque des revendications précédentes, **caractérisé en ce que,** dans l'état d'utilisation comprimé, ladite largeur (M) de l'élément de raidissement (6) à la transition entre la partie d'entre-jambes et la partie avant est comprise entre 20 et 35 mm.

5. Article selon l'une quelconque des revendications précédentes, **caractérisé en ce que,** dans l'état d'utilisation comprimé, ladite largeur (M) de l'élément de raidissement (6) à la transition entre la partie d'entre-jambes (3) et la partie avant (1) est comprise entre 25 et 30 mm.

6. Article selon l'une quelconque des revendications précédentes, **caractérisé en ce que** l'élément de raidissement (6) présente une rigidité à l'état sec comprise entre 1 et 15 N mesurée selon la norme ASTM D 4032-82.

7. Article selon l'une quelconque des revendications précédentes, **caractérisé en ce que** l'élément de raidissement (6) est constitué d'un mat de fibres formé à sec ayant une masse volumique comprise entre 0,15 et 0,75 g/cm3 et un poids par unité de surface compris entre 100 et 400 g/m2.

8. Article selon la revendication 7, **caractérisé en ce que** le mat de fibres formé à sec est, après la compression, ramolli mécaniquement à la rigidité souhaitée.

9. Article selon la revendication 7 ou 8, **caractérisé en ce que** le mat de fibres formé à sec est pourvu de la rigidité désirée et de l'extensibilité désirée en fonction du degré de compression choisi et au motif de compression choisi.

10. Article selon l'une quelconque des revendications précédentes, **caractérisé en ce que** les bords latéraux de l'élément de raidissement (6) qui, dans l'état d'utilisation comprimé de l'article, divergent au moins une portion du trajet à partir de la partie d'entre-jambes (3) sur la partie avant (1) de l'article, sont agencés dans ledit état de manière à former un angle (α) entre une ligne dans la direction longitudinale de l'article et chacun desdits bords latéraux de l'ordre de 35-55, de préférence de l'ordre de 45 °.

11. Article selon l'une quelconque des revendications précédentes, **caractérisé en ce que** l'élément de raidissement (6) s'étend également une portion du trajet sur la partie arrière (2) de l'article, et **en ce que** l'élément de raidissement est disposé si bien que ses bords latéraux (18, 19), dans ledit état d'utilisation comprimé de l'article, divergent dans la direction de la partie d'entre-jambes (3) au moins d'une portion du trajet à partir de la partie d'entre-jambes sur la partie arrière de l'article.

12. Article selon la revendication 11, **caractérisé en ce que** l'élément de raidissement (6) présente dans la partie arrière (2) une deuxième découpe (13) s'étendant à partir de son bord d'extrémité dans la direction vers la partie d'entre-jambes (3), en conséquence de quoi l'article est en cours d'utilisation muni d'un pli (17) le long de la direction longitudinale de l'article dans ladite deuxième découpe, ledit pli s'étendant dans la fente entre les fesses de l'utilisateur pendant l'utilisation de l'article.

13. Article selon la revendication 12, **caractérisé en ce que** la deuxième découpe (13) est cunéiforme et est située de façon symétrique et forme un angle (ß) compris entre 10° et 120°, préférablement entre 15° et 40°, à son extrémité faisant face à la partie d'entre-jambes.

14. Article selon la revendication 11, **caractérisé en ce que** l'élément de raidissement (6) présente dans la partie arrière (2) un deuxième trou de passage allongé (620) qui s'étend dans la direction longitudinale de l'article et le long de la ligne centrale de l'article, en conséquence de quoi l'article en cours d'utilisation est muni d'un pli le long de la direction longitudinale de l'article le long dudit deuxième trou, ledit pli s'étendant dans la fente entre les fesses de l'utilisateur pendant l'utilisation de l'article et de cette façon stabilisant l'article en position sur le porteur.

15. Article selon l'une quelconque des revendications précédentes, **caractérisé en ce que** l'élément de raidissement (6) présente une rigidité d'au moins 1,0 N, et **en ce que** l'élément de raidissement est conçu avec essentiellement la même rigidité sur toute l'étendue de l'élément de raidissement.

16. Article selon l'une quelconque des revendications précédentes, **caractérisé en ce que** l'élément de raidissement (6), vu dans la direction longitudinale de l'article, comprend un premier élément partiel avant de renfort (61) qui, dans l'état d'utilisation comprimé, présente ladite largeur (M) à la transition (12) entre la partie avant et la partie d'entre-jambes, et au moins un autre deuxième élément partiel de renfort (62) qui est séparé du premier élément partiel (61) au niveau de ladite transition (12), en conséquence de quoi le premier et le deuxième élément partiel peuvent être déplacés l'un par rapport à l'autre.

17. Article selon la revendication 16, **caractérisé en ce que** le deuxième élément partiel de renfort (62) s'étend également une portion du trajet sur la partie arrière (2) de l'article, et **en ce que**, dans l'état d'utilisation comprimé de l'article, les bords latéraux (18, 19) du deuxième élément de renfort sont disposés dans la direction de la partie d'entre-jambes (3) de manière à diverger d'au moins une portion du trajet à partir de la partie d'entre-jambes sur la partie arrière (2) de l'article.

18. Article selon la revendication 16 ou 17, **caractérisé en ce que** ladite deuxième découpe (13) est arrangée dans le deuxième élément partiel de renfort (62).

19. Article selon l'une quelconque des revendications précédentes, **caractérisé en ce que** l'élément de raidissement (6) s'étend au-dessus de ladite transition (12), et au moins une portion du trajet sur la partie avant (1) et également au moins une portion du trajet sur la partie d'entre-jambes, et **en ce que** la première découpe (610) est constituée d'au moins un trou qui est le plus large au moins juste en face de la transition (12) et est de forme circulaire ou oblongue.

20. Article selon l'une quelconque des revendications précédentes, **caractérisé en ce que** l'élément de raidissement (6) présente, à l'état plan avant l'utilisation, la même largeur le long de sa longueur à l'exception des parties d'extrémité qui sont convenablement arrondies.

21. Article selon la revendication 16, **caractérisé en ce que** la première découpe (610) est constituée d'une découpe ouverte en direction de la transition (12).

22. Article selon l'une quelconque des revendications 19 à 21, **caractérisé en ce que** la première découpe (610) est dans sa totalité située dans la partie avant (1) et est essentiellement en forme de losange.

23. Article selon l'une quelconque des revendications précédentes, **caractérisé en ce qu**'au moins un moyen élastique (16) est arrangé dans la direction longitudinale de l'article et au centre le long de la partie arrière de l'article et le long d'au moins une portion de celui-ci à partir de la partie d'entrejambes, ledit moyen élastique étant destiné, le long de sa longueur, à tirer des parties de matériau adjacentes pour les ramasser et à courber l'article vers le haut pour un meilleur contact avec le corps du porteur.

24. Article selon l'une quelconque des revendications précédentes, **caractérisé en ce que** l'élément de raidissement (6) sert de moyen d'absorption et présente une très grande capacité de dispersion des liquides pour étaler le fluide corporel reçu dans la zone d'entre-jambes (3) relativement étroite et délimitée par l'anatomie du porteur juste en face des parties génitales de l'utilisateur sur les parties absorbantes de l'article entier, et **en ce que** l'élément de raidissement est conçu avec une grande capacité de gonflement dans le sens de la profondeur et une grande capacité d'absorption accompagnante.

25. Article selon la revendication 24, **caractérisé en ce que** l'élément de raidissement (6) sert aussi d'élément d'absorption et est essentiellement homogène sur toute son étendue en ce qui concerne l'épaisseur, la rigidité, la capacité d'étalement et la capacité d'absorption, en conséquence de quoi la couche de raidissement et donc aussi l'élément d'absorption courbent uniformément pendant l'utilisation sans former irrégularités locales qui peuvent donner lieu à la diffusion indésirable de liquide.

26. Article selon l'une quelconque des revendications précédentes, **caractérisé en ce que** l'élément de raidissement (6) constitue aussi l'élément absorbant, et **en ce que** la forme de celui-ci et de la première découpe (610) est adaptée si bien que, lorsque l'élément de raidissement (6) est comprimé entre les tendons musculaires du porteur dans l'aine pendant l'utilisation de l'article, la largeur de l'élément de raidissement derrière ladite zone de transition (12) ne cesse de croître dans la partie d'entre-jambes dans la direction arrière vers la partie arrière (2) dans le but d'utiliser de façon optimale l'espace de largeur disponible dans cette zone en ce qui concerne l'absorption maximale.

27. Article selon l'une quelconque des revendications précédentes, **caractérisé en ce que** ledit élément de raidissement (6) est constitué d'au moins une zone comprimée d'un corps de matériau continu en une seule pièce.

28. Article selon l'une quelconque des revendications, **caractérisé en** ce **qu**'un élément formant une bosse (24) fait d'un matériau élastique est disposé sous l'élément de raidissement sur au moins une portion de la partie de l'entre-jambes (3), ledit élément formant une bosse étant agencé de façon à amener une partie surélevée (240) sur le côté qui est destiné à être adapté en forme contre le porteur, la partie surélevée étant agencée de façon à venir se situer directement devant les parties génitales de l'utilisateur après l'adaptation en forme de l'article sur le porteur.

29. Article selon la revendication 28, **caractérisé en ce que** la partie surélevée (240) est allongée dans la direction longitudinale de l'article et présente une longueur comprise entre 20 mm et 120 mm.

30. Article selon la revendication 28 ou 29, **caractérisé en ce que** la partie surélevée (240) est plus étroite que le reste de l'article dans la zone d'entrejambes, et **en ce que** la partie surélevée est plus épaisse que les zones environnantes.
